# EUROPEAN PATENT SPECIFICATION

(11) **EP 4 157 154 B1**
(45) Date of publication and mention of the grant of the patent: **28.01.2026**
(21) Application number: 21729305.9
(22) Date of filing: 28.05.2021
(51) Int. Cl.: A61F 2/30, A61L 27/18, A61L 27/44

(54) **MEDICAL IMPLANT FOR CARTILAGE REPLACEMENT AND METHOD OF MAKING SUCH IMPLANT**
MEDIZINISCHES IMPLANTAT FÜR KNORPELERSATZ UND VERFAHREN ZUR HERSTELLUNG EINES SOLCHEN IMPLANTATS
IMPLANT MÉDICAL POUR LE REMPLACEMENT DE CARTILAGE ET PROCÉDÉ DE FABRICATION D'UN TEL IMPLANT

(30) Priority: 29.05.2020 EP 20177539
(43) Date of publication of application: 05.04.2023
(73) Proprietor: DSM IP Assets B.V., 6221 BE Maastricht (NL); Academisch Ziekenhuis Maastricht, 6229 HX Maastricht (NL); Technische Universiteit Eindhoven, 5612 AE Eindhoven (NL); University Maastricht, 6211 LK Maastricht (NL)
(72) Inventor: OEVERING, Hendrik, 6100 AA ECHT (NL); GELISSEN, Franciscus Wilhelmus Maria, 6100 AA ECHT (NL); THIES, Jens Christoph, 6100 AA ECHT (NL); EMANS, Pieter, 6100 AA ECHT (NL); ROTH, Alex Kristian, 6100 AA ECHT (NL); VAN DONKELAAR, Corrinus Cornelis, 6100 AA ECHT (NL); ASIK, Emin Erkan, 6100 AA ECHT (NL)
(74) Representative: dsm-firmenich IP
(86) International application number: PCT/EP2021/064318
(87) International publication number: WO 2021/239932

(56) References cited:
- DE-A1- 10 156 610
- DE-A1- 3 917 035
- US-A1- 2004 188 011
- US-A1- 2009 043 398

## Description

### Field

The disclosed inventions relate to an orthopedic implant for use in resurfacing of a damaged joint surface, for example a cartilage replacement device, the implant having a bone anchoring part comprising a composite material, to a method of making the orthopedic implant, to a surgical kit comprising such implant, and to use of the implant and the kit in resurfacing a damaged joint surface. The closest prior art is document DE 3917035 A1, which defines the preamble of claim 1.

### Background

Orthopedic implants are medical implants used in orthopedic surgery concerning conditions that involve the musculosketetal system of a human or animal. This system provides for form, stability and movement of the body and is made up of the body's bones (the skeleton), muscles, cartilage, tendons, ligaments, joints, and other connective tissue (the tissue that supports and binds tissues and organs together). The musculoskeletal system's primary functions include supporting the body, allowing motion, and protecting vital organs. The joints and musculoskeletal tissues of the human body may be subject to traumatic injury, disease and degenerative processes that over a period of time can lead to deterioration or failure of a joint; causing severe pain or immobility. Generally, the ability of a joint to provide pain free articulation and carry load is dependent upon the presence of healthy bone, cartilage and associated musculoskeletal tissues that provide a stable joint. In connection with present disclosure orthopedic surgery also relates to maintaining the motion in the various joints of the human body. Orthopedic implants include devices used in partial or total joint arthroplasty, knee and hip prostheses, and osteochondral implants. Examples of orthopedic implants include bone anchors, plugs and screws, which are applied to fixate implants like artificial ligaments and tendons, meniscus or labrum replacement devices, as well as cartilage replacement devices.

Cartilage is a smooth, connective tissue on the surface of the ends of bone where they meet to form a joint, which protects and cushions bone, and absorbs forces transmitted throughout the body. Cartilage is an elastic tissue that permits smooth movement of joints, but it is without direct blood supply and has limited self-healing capacity in case of wear or trauma. A frequent and significant cartilage injury causing pain and/or immobility is damage to the articular cartilage in the knee, that is in the joint formed between the femur and tibia. Long-term, such initially local defect(s) may - if left untreated- lead to further degeneration and damaged cartilage in the joints and may require surgery using artificial prosthetic joints; like a partial or a total knee replacement (UKR / TKR, also called hemi / total knee arthroplasty or HKA / TKA). Such total replacement surgery, however, may be problematic as most artificial joints have limited durability and subsequently needed revision procedures are associated with longer operation and hospitalization times and may induce complications, especially in older patients. In order to delay and possibly even avoid the need for a joint replacement like a TKR, orthopedic implants have been developed to locally replace damaged cartilage; thereby creating a new smooth joint surface at the damaged site. Such implants are often referred to as cartilage plugs.

Such known *cartilage plugs,* also called osteochondral constructs, cartilage replacement devices, or resurfacing implants, are typically made from metals, such as titanium. The use of metal implants, however, leads to high rates of revision surgery, which might be related to the large difference in mechanical properties, like stiffness and deformability, between metal and (sub)chondral bone and cartilage tissue. Alternative devices made from natural and/or synthetic materials have been described or proposed in numerous publications. Cartilage plugs often have a cylindrical or a mushroom-like shape and may comprise at least two parts; a cartilage replacing part and a bone anchoring part. The cartilage replacement part may be typically made from a flexible, resilient and abrasion resistant biocompatible material that mimics some properties of natural cartilage, whereas the bone anchoring part may be made from a more rigid and stiff material, including metal.

One approach is to make a prosthetic plug from natural materials, including harvesting bone and cartilage from the patient (autogenous graft or autograft) and use of tissues from a genetically dissimilar donor of the same species (allograft or homograft). In US5782835, for example, an apparatus and method to implement such allograft approach is described. Use of such grafts, however, presents risks of infections or transmission of diseases.

Alternatively, orthopedic implants like a cartilage plug can be made from synthetic materials, like biocompatible polymers that may be biodegradable or biostable. Use of synthetic polymers would present advantages over metal implants, as polymers offer a large range of properties, may cause less damage to contacting tissue, and are not magnetic; thus more compatible with a medical imaging technique like MRI.

US2008/0249632A1 describes a cartilage plug that has a stepped-shoulder geometry comprising 4 or more layers, aiming at better distributing a load applied to the implanted plug and the surrounding tissue, and at reducing undesired movement. The different layers of the plug can be made from the same or from different materials, which may be selected from numerous natural and synthetic materials and may be porous or non-porous.

US2011/0218647A1 discloses a cartilage plug comprising a hydrogel, which contains a hydrophilic polymer, a fibrous filler and 40-80 mass% of water. The polymer is preferably crosslinked polyvinylalcohol. The implant would have a Young's modulus of 0.75-50 MPa, preferably 23-30 MPa, which enables initial compression to allow placing it in an opening and subsequent expansion for properly fitting.

US6626945B2 describes a cartilage plug that is formed of a laminated structure to match physiological requirements of the repair site. The plug may be of cylindrical shape and be formed from three different materials that are fused or bound together. In an embodiment a first layer, which upon implantation is closest to subchondral bone, is made from a biostable thermoplastic polyurethane (TPU) of Shore hardness 75 ShD. An intermediate layer of the implant is made from a TPU of 55 ShD hardness, whereas a third layer that is closest to the surface of the cartilage surrounding the implanted plug is made from a more flexible material like a TPU of hardness 80 ShA or from a thermoplastic hydrogel. This last layer is indicated to show properties similar to those of hyaline cartilage, which is the type of cartilage at the outer surface of an articulating joint; and the 75 ShD material would have an elastic modulus similar to subchondral bone.

WO2011/098473A1 describes orthopedic implants, like meniscus or spinal disc implants, which have two or more distinct sections that each comprise a different, but chemically related, polymeric material; such that sections are attached to each other at contact surfaces by interactions between said materials. Preferably the materials are chosen from blockcopolymers like thermoplastic polyurethanes (TPUs). The implants can be made by multi-component molding techniques. A meniscus prosthetic device is also described in WO2015/0135907A1, which comprises different parts made from two non-resorbable polymeric materials having a tensile modulus of at most 100 MPa and of at least 101 MPa, respectively. In experiments TPU materials with Shore hardness 80 ShA and 75 ShD were used. It is indicated that the material may be a polymer composition comprising TPU and up to 25 mass% of radiopaque filler particles like barium sulphate.

CN1215890C discloses polyurethane compositions which show anti-coagulation properties and which would be suited for making surgical products like sutures, catheters and prostheses. These compositions comprise 0.1-30 mass% of zinc oxide, titanium oxide or zirconium dioxide nanoparticles, with 80-99% of the particles having a size smaller than 50 nm.

In WO2007/007062A2 a cartilage repair implant is described wherein a resilient layer is bound to an anchor layer, which anchoring part is made from a bone cement composition that comprises an acrylate-based polymer containing calcium ions to facilitate bone ingrowth.

US2008/0081061A1 discloses an orthopedic device that comprises a composite material based on a biocompatible polymer having ceramic particles dispersed therein and may further include ceramic-free polymer integrally attached to the composite material. The ceramic material may be in particle or fiber form and may be selected, like the polymer, from extensive lists. In an embodiment the polymer is ultra-high molar mass polyethylene (UHMWPE) and the ceramic includes hydroxyapatite (HA) particles. Such composite part is made by mixing UHMWPE powder with (optionally coated) HA particles, and compression molding the mixture.

Geary et al. describe in Mater. Sci: Mater. Med (2008) 19:3355-3363 (DOI 10.1007/s 10856-008-3472-8) that thermoplastic polyurethanes, like commercially available Bionate^{®} polycarbonate urethane grades, have outstanding hydrolysis and ageing resistance; and are suitable biostable materials for use in making in vivo biomedical devices, for example devices for use in replacing diseased or damaged joints. Incorporating carbon fibers or hydroxyapatite (HA) particles in such polyurethanes via melt compounding may result in improved mechanical properties of the polymer material. It is also indicated, however, that such compounding step enhances polymer degradation resulting in significant reduction in molar mass of the polymer in the polymer composition. Especially in case of compounding polyurethane with HA, said degradation is shown to be more prominent than during a melt processing step like injection molding. In contrast to carbon fibers, a polyurethane composition based on TPU and HA particles did not show improved tensile properties.

In US2009/043398A1 a method of making an articulating surface implant such as a replacement plug is described, wherein a gradient in density, porosity and/or concentration is created in the implant by subjecting a viscous material to a centrifugal force; such as by spin casting. The viscous material may be a composite material comprising a polymer matrix and particles or fibers dispersed therein; the method resulting in an article having a concentration gradient in particles or fibers, and thus a gradient in stiffness.

US2004/0188011A1 discloses a method of making a prosthetic bearing element comprising a rigid backing made from carbon fiber reinforced polymer and which backing supports a soft elastomeric polyurethane bearing liner, in which method improved bonding of the backing to the bearing liner is achieved by laser welding; that is by passing laser light through the transparent bearing liner to cause heat fusion at the interface of the liner and the laser-opaque backing.

### Summary

Although various approaches have been proposed or described in literature, there still appears a need for an orthopedic implant comprising a bone anchoring part and a cartilage replacing part made from synthetic materials, which implant can provide a durable solution as replacement device for local cartilage defects in a joint, and which implant can be monitored during and after implantation with common medical techniques.

Objects of the present disclosure include providing such an orthopedic implant for use in resurfacing of a joint in case of locally damaged cartilage tissue, which implant comprises a bone anchoring part that enables a durable and monitorable connection to bone tissue.

The aspects and embodiments of the present inventions as described herein below and as defined in the claims provide an orthopedic implant that has a bone anchoring part showing good biocompatibility and mechanical properties compatible with bone tissue, and which implant can be monitored during and/or after implantation with medical imaging techniques like X-ray and MRI; and a method of making said implant. An aspect of the invention is thus an orthopedic implant as defined in claim 1.

The disclosed orthopedic implants, like cartilage replacement plugs, may have a substantially cylindrical or a mushroom-like shape and comprise at least two parts; said bone anchoring part and a cartilage replacing part that is typically made from a resilient and abrasion resistant biocompatible material. It was found that the relatively rigid anchoring part when comprising or substantially made from said TPU composition comprising zirconia particles allows inserting the implant into a pre-drilled bone hole to form a firm and durable connection to surrounding bone tissue, and which implant part and its connection to bone may be visualized or monitored over time with for example X-ray or MRI methods. The thermoplastic polyurethane composition shows favorable crystallization behavior and (mechanical) properties of the composition are better than those of similar compositions that are for example based on HA filler particles. Further advantages of using the thermoplastic polyurethane composition include freedom in designing and dimensioning of the implant, and in making the implant with common techniques like (multi-component) injection molding, allowing also more complex shapes. Especially when the implant comprises a cartilage replacing part made from a thermoplastic material compatible with the polyurethane-zirconia composition, like a more flexible or unfilled TPU composition, the implant may be made with a multi-component injection molding technique; which is a relatively simple method that generally results in sufficient adhesion between the bone anchoring and cartilage replacement parts without for example using an adhesive component.

In a further aspect, the invention relates to a method of making said orthopedic implant comprising a bone anchoring part, comprising forming the implant with a multi-component injection molding process comprising a step of molding the bone anchoring part from the polymer composition comprising a biostable thermoplastic polyurethane and 15-70 mass% of inorganic particles comprising zirconia.

In further aspects, the invention relates to an orthopedic implant comprising a bone anchoring part and a cartilage replacing part, wherein the bone anchoring part comprises a rigid polymer composition, and wherein a surface of the bone anchoring part that contacts the cartilage replacing part is structured, such as having protrusions and/or recessions of 0.05-3.0 mm; and to a method of making such an orthopedic implant.

In another aspect, the invention relates to a surgical kit of parts comprising at least one orthopedic implant of the invention, especially to a kit comprising a set of at least two implants having different sizes.

The invention may be used in the resurfacing of a joint that has locally degenerated or damaged cartilage tissue, like in a knee joint of a mammal. Such use for locally replacing damaged cartilage in a joint may delay and possibly even avoid the need for a total joint replacement surgery, like a TKR.

### Brief description of Figures

The invention will be further elucidated by the following illustrative figures, without being restricted thereto. In the Figures like numbers represent like elements.
Figure 1 schematically depicts a 2-part cylindrical cartilage plug.
Figures 2A and 2B schematically show a mushroom-like cartilage plug in two different views.
Figure 3 shows an angled view of a part of a biplanar contoured cartilage replacing part 2 of uniform thickness.
Figure 4 schematically depicts a cross-sectional view of a mushroom-like plug comprising a bone anchoring part having two 'stems' (3b' and 3b").
Figures 5A to 5C schematically depict different views of a cartilage plug comprising a bone anchoring part having two 'stems' and a cartilage replacing part having a contoured surface.
Figures 6A-6C show schematical drawings of cartilage plugs comprising a bone anchoring part (3a and 3b) and a (transparent) cartilage replacing part (2), wherein the surface of 3a is smooth (Fig. 6A), structured with ridges (Fig. 6B) or structured with multiple small protrusions (Fig. 6C).
Figure 7 shows particle size distributions as measured on 3 grades of zirconia.
Figure 8 represents DSC curves (heating-cooling-reheating) as obtained on unfilled polyurethane and on corresponding polyurethane compositions containing 20, 40 and 60 mass% of zirconia (SA).
Figure 9 provides scoring of Bone to Implant Contact (BIC, %) as an indication of osseointegration for implanted plugs M (metal), U (unfilled polyurethane) and I (invention; zirconia-filled polyurethane, BCP-coated), after 6 months of being implanted in goat knees.
Figures 10A to 10C show representative micrographs of histological slices taken from goat-implanted plugs M, U and I (after 6 months).
Figure 11 presents Modified Mankin score (MMS) results of articulating cartilage quality on the opposing bone surface of a joint with implanted plugs M, I and U as compared with a sham-operated joint (after 6 and 12 months).

### Detailed Description

Within the context of present disclosure, a *biocompatible* material is biologically compatible by not producing a toxic, injurious, or immunologic response when in contact with living tissue. *Biodegradable* means a material is susceptible to chemical degradation or decomposition into simpler components under physiological conditions or by biological means, such as by an enzymatic action. *Biostable* or bioinert means that the material is substantially non-biodegradable under conditions and time of intended use in contact with living tissue.

In accordance with an aspect, the invention provides an orthopedic implant, for example a biostable implant suitable for use in repair of damaged cartilage tissue, the implant having a bone anchoring part comprising a polymer composition comprising a biostable thermoplastic polyurethane and 15-70 mass% of inorganic particles comprising zirconia.

The polymer composition in the bone anchoring part comprises a biostable thermoplastic polyurethane (TPU). A thermoplastic polyurethane is a non-crosslinked polyurethane, which has essentially linear polymer chains, is soluble in a good solvent and can be melted by increasing temperature and be re-solidified upon subsequent cooling; allowing melt processing via e.g. extrusion or injection molding. Thermoplastic polyurethanes are typically block copolymers (also called segmented copolymers).

Block copolymers are polymers comprising multiple blocks (also called segments) of polymers (including oligomers) that are chemically distinct, and which show different thermal and mechanical properties, and different solubilities. Often the blocks in a block copolymer comprising two (or more) types of blocks are referred to as being 'hard' and 'soft' polymer blocks, such (chemically) different blocks resulting in microphase separation into domains that are rich in either hard or soft blocks. The hard block in a block copolymer typically comprises a rigid or high modulus polymer, with a melting temperature (Tₘ) or a glass transition temperature (T_{g}) higher than the use temperature, typically of about 35-40 °C. The soft blocks in the block copolymer generally comprise a flexible, low modulus, amorphous polymer with a T_{g} lower than 25 °C, preferably lower than 0 °C. As for most mechanical properties, thermal parameters like Tₘ and T_{g} are generally determined on dry samples; using well-known techniques like DSC or DMA. In such phase-separated block copolymers, the hard segments function as physical (and non-permanent) crosslinks for the flexible soft segments, resulting in materials having properties ranging from fairly stiff and rigid to flexible and elastic, depending e.g. on the ratio of hard to soft blocks. Depending on type and content of hard blocks, the polyurethane block copolymer may show good stability and elasticity over a desired temperature range without the need for chemical crosslinking; and can generally be processed as a thermoplastic. The term thermoplastic polyurethane basically denotes a family of polymers with a backbone comprising the reaction product of at least three principle components; which are a diisocyanate, a diol chain extender and a polymer diol or macroglycol. Optionally, a monofunctional compound may be used as a further component functioning as a chain stopper and forming (non-reactive) endgroups. In embodiments, the backbone of the polyurethane or TPU applied in present invention is substantially linear.

In embodiments, the TPU comprises hard blocks that include urethane groups and optionally urea groups in repeating units, which groups have resulted from reaction of a diisocyanate with a diol and optionally a diamine as respective chain extenders.

Suitable diisocyanates include aromatic, aliphatic and cycloaliphatic compounds, having an average of 1.9-2.1 isocyanate groups per molecule. In an embodiment, the diisocyanate comprises 4,4'-diphenylmethane diisocyanate (MDI), 2,4-toluene diisocyanate, 2,6-toluene diisocyanate (TDI), 1,4-phenylene diisocyanate, hexamethylene diisocyanate (HDI), tetramethylene-1,4-diisocyanate, cyclohexane-1,4-diisocyanate, dicyclohexylmethane-4,4'-diisocyanate (CHMDI), isophorone diisocyanate (IPDI), or a mixture thereof. In an embodiment, the diisocyanate comprises hexamethylene diisocyanate, dicyclohexylmethane 4,4'-diisocyanate, isophorone diisocyanate, or a mixture thereof. In an embodiment, the diisocyanate consists of hexamethylene diisocyanate, dicyclohexylmethane 4,4'-diisocyanate, isophorone diisocyanate, or a mixture thereof. In another embodiment, the diisocyanate comprises 4,4'-diphenylmethane diisocyanate, 2,4-toluene diisocyanate, 2,6-toluene diisocyanate, or 1,4-phenylene diisocyanate. In an embodiment, the diisocyanate consists of 4,4'-diphenylmethane diisocyanate, 2,4-toluene diisocyanate, 2,6-toluene diisocyanate, 1,4-phenylene diisocyanate, or a mixture thereof. In an embodiment, the molar mass of the diisocyanate is from 100 to 500 g/mol. In an embodiment, the molar mass of the diisocyanate is from 150 to 260 g/mol.

Chain extenders are typically low molar mass aliphatic compounds, having two or more, preferably two, hydroxyl or amine groups. Bifunctional chain extenders result in linear, generally thermoplastic polymers, whereas multifunctional chain extenders and/or isocyanates would lead to branched or cross-linked products. In an embodiment, the bifunctional chain extender has a molar mass of at least 60 g/mol, at least 70 g/mol, at least 80 g/mol, at least 90 g/mol, or at least 100 g/mol. In an embodiment, the chain extender has a molar mass of at most 500 g/mol, at most 400 g/mol, at most 300 g/mol, at most 200 g/mol, or at most 150 g/mol. In an embodiment, the chain extender comprises ethylene glycol, diethylene glycol, propylene glycol, dipropylene glycol, 1,3-propanediol, 1,4-butanediol, 1,5-pentanediol, 1,6-hexanediol, or 1,8-octanediol; and/or such corresponding diamines. In embodiments, the thermoplastic polyurethane comprises only diol chain extenders.

In other embodiments, the TPU comprises hard blocks having both urethane and urea linkages. The advantage thereof is enhanced interaction between the hard blocks, resulting in higher softening temperature and/or allowing a higher content of soft blocks; resulting in block copolymers showing enhanced flexibility and elasticity, and excellent flex life or fatigue resistance. Depending on the ratio diol/diamine, the polyurethane chains may show such strong interactions that at a melt processing temperature thermal degradation may occur to such extend that solution processing is to be preferred for optimal performance. Commercially available examples of such polyurethanes comprising both urethane and urea linkages, sometimes also referred to as polyurethane ureas, include Biospan^{®} products (available from e.g. DSM Biomedical BV, Sittard-Geleen NL).

In further embodiments, the thermoplastic polyurethane comprises soft blocks derived from at least one aliphatic polymer diol or polyol, which is chosen from the group consisting of polyethers, polyesters, polyacrylates, polyolefins and polysiloxanes (also called silicones); which polymers are bifunctional with hydroxyl (or amine) terminal groups. Such polymer diols for the soft blocks are understood herein to include oligomers, homopolymers and copolymers, and polyesters are considered to include polycarbonates. Generally known polyurethane block copolymers and methods to prepare these copolymers are described in a.o. US4739013, US4810749, US5133742 and US5229431.

In embodiments of the present disclosure the thermoplastic polyurethane comprises in soft blocks at least one polymer diol chosen from an aliphatic polyester diol, aliphatic polycarbonate diol, an aliphatic polyether diol, a poly(isobutylene) diol and a polysiloxane diol. Like or chain extenders, also some amine-functional soft blocks can be used, resulting in additional urea linkages. Biocompatibility and biostability of such polyurethane block copolymers in the human body has been proven.

Mechanical and other properties of a thermoplastic polyurethane can be tailored by varying chemical compositions and/or molar masses of the blocks. The hard blocks of a thermoplastic polyurethane contained in the composition of the invention may have a molar mass of about 160 to 10,000 Da, and more preferably of about 200 to 2000 Da. The molar mass of the soft segments may be typically about 200 to 100,000 Da, and preferably at least about 400, 600, 800 or 1000 Da and at most about 10,000, 7500, 5000, 4000, 3000 or 2500 Da. Within the context of present disclosure, molar mass of polymers and oligomers forming blocks in the polymer refers to the number average molar mass (Mₙ), as for example derived from GPC measurements. The ratio of soft to hard blocks can be chosen to result in certain stiffness or hardness of the polyurethane. Typically, hardness of the polyurethane as measured with the Shore durometer hardness test using A or D scales, may be from 40 ShA up to 90 ShD, generally representing a flexural modulus range of about 10 to 2000 MPa. In embodiments, the thermoplastic polyurethane has a hardness from 45 ShA to 90 ShA, preferably at least 50, 55 or 60 ShA. The advantage of using a TPU of relatively low hardness may be a higher toughness of the resulting composition further comprising zirconia. In other embodiments the TPU in the polymer composition has a hardness of from 90 ShA to 90 ShD, as this will result in higher stiffness of the composition. In further embodiments, the TPU has a hardness of at least 40, 50 or 60 ShD and of at most 85 or 80 ShD, for a good balance between stiffness and processing behavior.

In further embodiments of present invention, the thermoplastic polyurethane comprises an aliphatic polyether, an aliphatic polyester or an aliphatic polycarbonate in the soft blocks, more specifically an aliphatic polycarbonate. Compositions of soft blocks are preferably chosen such that an essentially amorphous oligomer or polymer having a T_{g} below 10, 0, and preferably below -10 °C results. Suitable aliphatic polyethers include poly(propylene oxide) diols, poly(tetramethylene oxide) diols, and their copolymers. Suitable aliphatic polyesters are generally made from at least one aliphatic dicarboxylic acid and at least one aliphatic diol. Aliphatic polycarbonate diols are based on similar aliphatic diols as used for polyester diols, and can be synthesized via different routes as known in the art. Suitable examples include poly(hexamethylene carbonate) diols and poly(tetrahydrofuran carbonate) diols. Such polycarbonate-based TPUs show favorable biocompatibility like hemocompatibility, and enhanced biostability. In an embodiment, the soft block of the TPU is based on a poly(hexamethylene carbonate) diol, a poly(polytetrahydrofuran carbonate) diol, or a mixture thereof. In a preferred embodiment, the TPU comprises, or is substantially based on a poly(hexamethylene carbonate) diol in soft blocks.

In further embodiments, the soft block of the TPU comprises a polysiloxane diol such as a poly(dimethyl siloxane) diol, a polycarbonate diol, or a poly(tetramethylene oxide) diol. In an embodiment, the soft block is based on a polysiloxane diol, a polycarbonate diol, a poly(tetramethylene oxide) diol, or a mixture thereof. In an embodiment, the soft block comprises a mixture of two or more of a polysiloxane diol, a polycarbonate diol, or a poly(tetramethylene oxide) diol. Such mixtures allow making biocompatible polyurethanes that show enhanced hydrolytic stability combined with high toughness. In an embodiment, the soft block is based on a mixture of two or more of a polysiloxane diol, a polycarbonate diol, or a poly(tetramethylene oxide) diol. In an embodiment, the soft block comprises a polysiloxane diol and one or more of a polycarbonate diol and a poly(tetramethylene oxide) diol. In an embodiment, the soft block is based on a polysiloxane diol and one or more of a polycarbonate diol and a poly(tetramethylene oxide) diol.

In an embodiment, the soft blocks may further comprise a C₂-C₁₆ fluoroalkyl diol or C₂-C₁₆ fluoroalkyl ether diol. In an embodiment, the soft block in the polyurethane backbone comprises the residue of 1H,1H,4H,4H-Perfluoro-1,4-butanediol, 1H,1H,5H,5H-Perfluoro-1,5-pentanediol, 1H,1H,6H,6H-perfluoro-1,6-hexanediol, 1H,1H,8H,8H-Perfluoro-1,8-octanediol, 1H,1H,9H,9H-Perfluoro-1,9-nonanediol, 1H,1H,10H,10H-Perfluoro-1,10-decanediol, 1H,1H,12H,12H-Perfluoro-1,12-dodecanediol, 1H,1H,8H,8H-Perfluoro-3,6-dioxaoctan-1,8-diol, 1H,1H,11H,11H-Perfluoro-3,6,9-trioxaundecan-1,11-diol, fluorinated triethylene glycol, or fluorinated tetraethylene glycol.

In an embodiment, the C₂-C₁₆ fluoroalkyl diol or C₂-C₁₆ fluoroalkyl ether diol has an Mₙ of at least 150 g/mol, at least 250 g/mol, or at least 500 g/mol. In an embodiment, the fluoroalkyl diol or fluoroalkyl ether diol has a molar mass of at most 1500 g/mol, at most 1000 g/mol, or at most 850 g/mol. In an embodiment, the C₂-C₁₆ fluoroalkyl diol or C₂-C₁₆ fluoroalkyl ether diol is present in an amount of at least 1 mass%, at least 2 mass%, or at least 5 mass%, based on the total mass of the polyurethane. In an embodiment, the C₂-C₁₆ fluoroalkyl diol or C₂-C₁₆ fluoroalkyl ether diol is present in an amount of at most 15 mass%, at most 10 mass%, or at most 8 mass%, based on the total mass of the polyurethane.

In embodiments, the polyurethane may comprise one or more hydrophobic or hydrophilic endgroups. An endgroup is a generally a non-reactive moiety present at a terminal end of a molecule. In an embodiment, the polyurethane comprises an endgroup at each terminus of the backbone; i.e. it has an average of about 2 endgroups. In an embodiment, the endgroup is a linear compound. In another embodiment, the endgroup is branched. An endgroup may have been formed by reacting a terminal isocyanate group present during or after forming the polymer backbone with a co-reactive group on a monofunctional compound, also called chain stopper. For instance, a formulation for forming a polyurethane may comprise a diisocyanate, a polymeric aliphatic diol, a chain extender, and a monofunctional alcohol or amine; like 1-octanol or octylamine to form a C₈ alkyl endgroup.

In embodiments, the endgroup is a hydrophobic endgroup, for example comprising a C₂-C₂₀ alkyl, a C₂-C₁₆ fluoroalkyl, a C₂-C₁₆ fluoroalkyl ether, a hydrophobic poly(alkylene oxide) or a polysiloxane, including copolymers thereof. In an embodiment, the hydrophobic poly(alkylene oxide) is poly(propylene oxide), poly(tetramethylene oxide) or a copolymer thereof. In an embodiment, the hydrophobic endgroup is a polysiloxane, like a poly(dimethyl siloxane). In an embodiment, the endgroup comprises C₂-C₂₀ alkyl, C₂-C₁₆ fluoroalkyl, C₂-C₁₆ fluoroalkyl ether, or a hydrophobic poly(alkylene oxide). Such endgroups may be formed with monofunctional alcohols, including carbinols, or amines of the foregoing. Such polyurethane elastomers having hydrophobic endgroups are found to positively affect properties of the polyurethane and its interaction with other materials, including other polymers like polyolefins and bodily tissue and fluid like blood.

In an embodiment, the hydrophobic endgroup comprises C₂-C₁₆ fluoroalkyl or C₂-C₁₆ fluoroalkyl ether. Such endgroups may be formed with monofunctional alcohols or amines comprising C₂-C₁₆ fluoroalkyl or C₂-C₁₆ fluoroalkyl ether. In an embodiment, the endgroup is formed from 1H, 1H-Perfluoro-3,6-dioxaheptan-1-ol, 1H, 1H-Nonafluoro-1-pentanol, 1H,1H-Perfluoro-1-hexyl alcohol, 1H,1H-Perfluoro-3,6,9-trioxadecan-1-ol, 1H,1H-Perfluoro-1-heptyl alcohol, 1H, 1H-Perfluoro-3,6-dioxadecan-1-ol, 1H,1H-Perfluoro-1-octyl alcohol, 1H, 1H-Perfluoro-1-nonyl alcohol, 1H,1H-Perfluoro-3,6,9-trioxatridecan-1-ol, 1H, 1H-Perfluoro-1-decyl alcohol, 1H, 1H-Perfluoro-1-undecyl alcohol, 1H, 1H-Perfluoro-1-lauryl alcohol, 1H, 1H-Perfluoro-1-myristyl alcohol, or 1H,1H-Perfluoro-1-palmityl alcohol.

In another embodiment, the endgroup is a hydrophilic endgroup that is formed from a hydrophilic monofunctional alcohol or amine compound. Such compound is typically soluble in water and may show surface activity; like a polyethylene oxide or a sulfonate-functional compound. Such hydrophilic endgroups may affect interactions with other materials, for example enhance dispersion of certain inorganic filler particles.

In another embodiment, the polyurethane comprises a mixture of hydrophobic and hydrophilic endgroups. Such modification allows to control the hydrophobic vs hydrophilic balance of the polymer. A general advantage of using TPUs having endgroups is to modify and control properties of the polymer without incorporating additives that may introduce potential problems with migration from the polymer and implant.

In an embodiment, the endgroup is monomeric and has a molar mass of 200 g/mol or more, 300 g/mol or more, or 500 g/mol or more; and of 1,000 g/mol or less or 800 g/mol or less.

In other embodiments, the endgroup is polymeric and has a molar mass of 10,000 g/mol or less, 8,000 g/mol or less, 6,000 g/mol or less, or 4,000 g/mol or less. In an embodiment, the endgroup is polymeric and has a molar mass of 500 g/mol or more, 1,000 g/mol or more, or 2,000 g/mol or more.

In an embodiment, the endgroup is present in an amount of at least 0.1 mass%, at least 0.2 mass%, at least 0.3 mass%, or at least 0.5 mass%, based on the total mass of the polyurethane. In an embodiment, the endgroup is present in an amount of at most 3 mass%, at most 2 mass% or at most 1 mass%, based on the total mass of the polyurethane. In an embodiment, the endgroup is present in an amount of at least 0.1 mass%, at least 0.2 mass%, at least 0.3 mass%, or at least 0.5 mass%; and in an amount of at most 3 mass%, at most 2 mass% or at most 1 mass%, based on the total mass of the polyurethane.

In an embodiment, the polyurethane comprises less than 0.1 mass% of endgroups, based on the total weight of the polyurethane. In an embodiment, the polyurethane is substantially devoid of endgroups. In an embodiment, the polyurethane is devoid of endgroups.

The hard blocks in the TPU are typically based on an aromatic diisocyanate like toluene diisocyanate (TDI) or methylenediphenyl diisocyanate (MDI), and a low molar mass aliphatic diol like 1,4-butanediol. Polyether and polycarbonate polyurethanes may be suitably used for biomedical applications, in view of their flexibility, strength, biostability, biocompatibility and wear resistance. A TPU containing a combination of a polyether and a polysiloxane or a polycarbonate and a polysiloxane in the soft blocks shows a unique combination of properties and may advantageously be used as the polyurethane in the polymer composition. Commercially available examples of such polymers include Pursil^{®} and Carbosil^{®} products (available from DSM Biomedical BV, Sittard-Geleen NL).

In a further embodiment, the TPU may be a blend of two or more polymers, for example a blend of two biocompatible TPU grades having at least different hardness, like a combination of 80 ShA and 75 ShD grades.

In other embodiments the TPU may comprise one or more customary additives that are allowed for the targeted use of the TPU and the polymer composition in an implant; in addition to e.g. catalyst residues. Examples of additives include stabilizers, anti-oxidants, processing aids, lubricants, surfactants, antistatic agents, colorants, etc.. The additives may be present in the typically effective amounts as known in the art, such as 0.01-5 mass% based on the amount of the polyurethane, preferably 0.1-2 mass%. In another embodiment, the TPU is substantially free of additives.

In embodiments, the polymer composition substantially consists, or consists of a) 30-85 mass% of a biostable thermoplastic polyurethane and b) 15-70 mass% of zirconia particles, with the sum of a) and b) being 100 mass%.

In other embodiments, the polymer composition consists of a) 20-85 mass% of a biostable thermoplastic polyurethane, b) 15-70 mass% of zirconia particles, and c) 0-10 mass% of other compounds, with the sum of a) and b) being 100 mass%. Examples of other compounds include bioactive compounds like anti-microbial or anti-inflammatory agents, an active agent or drug that reduces pain or improves healing or bone formation; or dispersion agents or other compounds purposely added, including residual amounts of solvents that may for example have been used in making the composition, including for cleaning equipment used. Said (bio-)active compounds may be present as such in the composition or as a formulation that controls release of active compound to surrounding tissue. Such other compounds are generally present in relatively small amounts, for example from about 0.1 to 5 mass% based on total polymer composition. In embodiments, the composition -and parts made therefrom-comprise at most 5, 4, 3 or 2 mass% of other compounds, and at most 1000 ppm of solvent, preferably at most 800, 600, 500 or 400 ppm of solvent.

The polymer composition that is contained in the bone anchoring part comprises 15-70 mass% of inorganic particles comprising zirconia. Zirconia is also referred to as zirconium dioxide or ZrO₂, a white crystalline oxide of zirconium. Zirconia is known to be radiopaque and may add radiopacity to an organic polymer by mixing a certain amount of particulate material into such matrix.

In embodiments, the inorganic particles in the polymer composition substantially consist or consist of zirconia.

In embodiments, zirconia particles form at least 60, 70 or 75 mass% and at most 98, 95, 90, 85 or 80 mass% of the total amount of inorganic particles in the polymer composition.The main use of zirconia is in the production of hard ceramics, for example by sintering at high temperature. Within the biomedical field such ceramic use is typically in dentistry as crowns and bridges. Other uses include protective, optical and thermal barrier coatings, ceramic knives and as diamond simulant in jewelry. Unlike many other mineral particles, zirconia is seldomly used as a filler or reinforcing agent in thermoplastic polymer compositions (also referred to as polymer compounds).

Zirconia as such is chemically stable, although it may undergo phase changes at high temperatures; that is at temperatures much higher than during compounding with a thermoplastic polyurethane, during injection molding of the present composition or during use of an implant made therefrom. Commercially available zirconia grades may contain other elements as dopants to thermally stabilize certain phases; like MgO, Y₂O₃, CaO or Ce₂O₃ added in amounts from 1 to more than 10 mol%. In addition, zirconia grades may comprise small amounts of elements like Al, SI, Fe, Na. Within the context of this disclosure, zirconia is therefore understood to comprise substantially pure ZrO₂ as well as mixed oxides comprising ZrO₂ and up to about 20 mass% of other inorganic oxides, preferably at most 15, 10 or 5 mass%.

In other embodiments, the inorganic particles in the polymer composition are radiodense, biocompatible transition metal compound particles, like transition metal salt particles or preferably transition metal oxide particles as inorganic particles.

In embodiments, the biocompatible transition metal compound particles comprise a salt, preferably an oxide of at least one of titanium (Ti), zinc (Zn), yttrium (Y), zirconium (Zr), lanthanium (La), ytterbium (Yb), hafnium (Hf), and tantalum (Ta). In other embodiments, the inorganic particles comprise a salt, preferably an oxide of at least one of Ti, Zn, Y, Zr, and Ta. Alternatively, the polymer composition comprises inorganic particles substantially consisting of one or more of said salts or oxides.

In embodiments, the polymer composition comprises inorganic particles comprising an oxide of titanium, preferably the particles substantially consist or consist of titania, Titania is also referred to as titanium dioxide or TiO₂. Titania occurs in different mineral forms, like rutile and anatase, and is mostly used as a white pigment in paints, plastics, foods, toothpastes and pills.

In embodiments, the polymer composition comprises particles comprising an oxide of zinc, preferably the polymer composition comprises particles that substantially consist or consist of zinc oxide (ZnO). ZInc oxide as occuring in nature typically contains a number of impurities, and is therefore generally synthesized from metallic zinc. Pure zinc oxide is a white powder that is applied in many different applications, including paints, as filler in plastics, rubber and cements, in ceramics, and in dentistry and skin care products (in view of its anti-bacterial properties).

A part made from the polymer composition comprising polyurethane, zirconia and optionally other inorganic particles and other compounds, is radiopaque and MRI compatible, and such implant part may thus be distinguished from other material and tissue with medical X-ray techniques. Radiopacity or radiodensity, and radiopaque or radiodense means that a part comprising such inorganic particles inhibit passage of (or absorb) the radio-wave and X-ray portion of the electromagnetic spectrum to such extend that enough contrast with natural tissue is visible with medical X-ray imaging techniques (also called radiographic imaging). Factors contributing to radiopacity are electron density and atomic number. MRI compatibility or compatible means the material poses no known hazards in all MRI environments; i.e. it is nonconducting, non-metallic and non-magnetic. Zirconia is not magnetic, and poses no known hazards in MRI environments. Addition of zirconia to polyurethane thus also enables imaging a part made from the present composition with MRI techniques. An implant made from the composition may thus be visualized using such common medical imaging techniques as X-ray and MRI; enabling monitoring during surgical procedures to properly position the implant at a targeted implant site as well as post-operative inspection of its position in relation to surrounding tissues.

The inorganic particles including zirconia in the polymer composition typically have an average particle size in the range 0.03 - 10 µm, preferably 0.1-5 µm. Within present disclosure particle size of the inorganic particles is the D₅₀ value, i.e. the median diameter or the medium value of particle size distribution, as measured with light diffraction according to ISO 13320:2009, e.g. with a Malvern Mastersizer 2000. Particle size as measured relates to size of the particles as dispersed in water, which may be different from particle size distribution in a polymer composition; as primary particles may not de-agglomerate or disperse in the same way as during mixing with (more viscous) polymer.

The inorganic particles in the polymer composition may have different types of particle shape, and may be of regular or of irregular form. Particle shape may range from virtually spherical to more elongated or flat shapes; like a cigar-, platelet-, needle- or fiber-like shape; with a cross-section that can be round, oval, triangular, rectangular, square, and with an average aspect ratio of at least 1, 2, 4, 6, 8 or 10. An advantage of substantially round particles may be isotropic properties of the composition, whereas elongated shapes may result in compositions with better mechanical properties, but which may depend on orientation of said particles. In embodiments, the polymer composition comprises a mixture of particles of different shapes, like a mixture of virtually spherical particles and elongated particles.

In embodiments the particles have a size D₅₀ of at least 0.05, 0.10, 0.15, 0.20, 0.25 or 0.30 µm, as handleability, like flow and dosing behavior, generally improves with particle size. In view of dispersibility in polyurethane and mechanical properties of the polymer composition, particle size of inorganic particles is at most 8, 7, 6, 5, 4, 3 or 2 µm. The inorganic particles in the composition may also be a mixture of different sizes, like a mixture of particles having a size in the lower end and a size in the higher end of said ranges; for example to optimize density and mechanical properties like stiffness or toughness of the composition.

In embodiments, the polymer composition comprised in the bone anchoring part contains a biostable thermoplastic polyurethane and 15-70 mass% of inorganic particles comprising zirconia particles. A relatively high amount of such particles will enhance rigidity, for example tensile modulus, of a part made from the composition, but may deteriorate extensibility and toughness of the composition. Also, polymer degradation due to shear during compounding to make the composition may be enhanced by high amounts of particles. In embodiments, the composition therefore contains at least 20, 25, 30, or 35 mass% of inorganic particles and at most 68, 66, 64, 62, 60, 58, 56, 54, 52, 50, 48 or 46 mass%.

In embodiments, the polymer composition in the bone anchoring part contains 15-70 mass% of a combination of zirconia particles and one or more other biocompatible inorganic particles, including other transition metal compound particles as defined above and/or natural mineral particles like (nano)clay, mica, talcum, etc., and which may be dispersed in the polyurethane. Other inorganic particles typically have a similar particle size range as the zirconia; but may also be of different size and have a different particle shape, to result in a combination of for example spherical and needle- or fiber-like particles. Presence or fiber-like or other elongated particles may enhance mechanical properties of the polymer composition. The other particles may be inert or show bioactivity like osteoconductivity; like bioglass or other silicated bioceramics. In embodiments, at most 40 mass% of the total amount of zirconia and other filler particles that is present in the polymer composition is formed by the other inorganic particles, preferably at most 30, 25, 20, 15, 10 or 5 mass%.

In further embodiments, the polymer composition is substantially free from particles based on calcium phosphates like hydroxyapatite, in view of potential enhanced degradation as reported in literature, and as observed upon making such polyurethane compositions (see experiments). More generally, in embodiments the composition is substantially free from inorganic particles or other compounds that may initiate or enhance (hydrolytic) degradation of polyurethane, because such particles or compounds comprise reactive groups that promote such degradation or have such hygroscopic properties that they cannot be suitably dried to a sufficiently low moisture content; such as to below 250, 150 or 100 ppm.

The polymer composition comprised in the bone anchoring part can be made using different mixing equipment and processes, as are known by a skilled person; for example by using a solvent-assisted mixing process or a melt-mixing process.

In an aspect, the invention provides a method of making the polymer composition, as described above including all variations and preferred embodiments and combinations thereof, comprising steps of
- Providing a biostable thermoplastic polyurethane with a moisture content of at most 300 ppm;
- Providing inorganic particles comprising zirconia having a moisture content of at most 250 ppm;
- Optionally mechanically or chemically treating the inorganic particles;
- Optionally providing other compounds having a moisture content of at most 250 ppm; and
- Mixing the polyurethane, the inorganic particles and optional other compounds.

In general, the polyurethane polymer and any zirconia or other inorganic particles to be added are thoroughly dried before mixing. Typically, the polyurethane is dried for several hours, e.g. from 4 to 30 h, at temperatures below its softening or melting point to obtain a moisture level of at most 300 ppm, preferably at most 250, 200 or 150 ppm. Zirconia and other particles may be dried at relatively high temperature during prolonged time, for example at 100-200 °C during 20-40 h, to obtain a moisture level of at most 250 ppm, preferably at most 150 or 100 ppm.

In embodiments, the method comprises a step of mechanically or chemically (pre)treating the (dried) inorganic particles, to further enhance properties of the resulting polymer composition. In embodiments, mechanically treating comprises milling or grinding the particles, optionally in the presence of an auxiliary component. Such auxiliary component may be a low viscosity or a viscous liquid, a dispersion aid and/or a polymer, each being biocompatible and compatible with the polyurethane. Such treatment may be facilitated with sonication to break down aggregates of particles and/or enhance dispersion. Such mechanical treatment step may result in a powder, a dispersion, a paste or a solid composition comprising the inorganic particles, use of which may result in an improved dispersion level of the particles in polyurethane in subsequent mixing step. The treated inorganic particles may be dried to arrive at a moisture level of at most 250 ppm.

In further embodiments, the method comprises a step of chemically treating the (dried) zirconia and optional inorganic particles, to change the type and/or amount of functional groups at the surface of the particles; and therewith dispersibility in and/or interfacial adhesion of the particles to polyurethane. The treatment may include a corona treatment, a plasma treatment, and/or a wet chemical treatment. A corona or plasma treatment as such can modify the surface of the particles as is known to a skilled person, but may also be combined with wet chemical modification. In a wet chemical treatment, the particles are typically first dispersed in a suitable liquid, and then a reagent is added, generally in an amount of 5-300 mass% relative to particles. Dispersion may be facilitated with sonication to break down aggregates of particles. Suitable reagents are for example organic compounds having alkyl, amine, carboxylic or peroxide functionality, or silane compounds. Examples include carbon dioxide, oxygen, unsaturated hydrocarbons, alkyl amines, carboxylic acids, and various amine- and/or alkoxy-functional silanes like 3-aminopropyltriethoxylsilane. Such compounds are also referred to as coupling agents in the art. In view of targeted medical use of the polymer composition, addition of other components like a catalyst is preferably omitted and un-reacted reagents and liquid are substantially fully removed after the pretreatment, and moisture level is brought to at most 250 ppm. The skilled person will be able to select a reagent and suitable treatment conditions, based on general knowledge and optionally some routine experiments.

In embodiments, other compounds as described above for the polymer composition may be provided, which compounds have a moisture content of at most 250 ppm. Examples of other compounds include bioactive compounds, stabilizers and compounds that aid in dispersing the inorganic particles in the TPU during mixing.

In embodiments, the method of making the polymer composition comprising a biostable thermoplastic polyurethane and 15-70 mass% of inorganic particles comprises adding dried (zirconia) particles or a dried masterbatch comprising such particles during synthesis of the polyurethane. For example, such addition can be done before a 1-step polymerization reaction by mixing with, for example liquid, starting chemicals, or during a 2-step polymerization, e.g. by mixing particles with prepolymer before or during a second polymerization step. In other embodiments, the method of making the polymer composition comprising a biostable thermoplastic polyurethane and inorganic particles comprises providing a polymerized polyurethane and using a solvent-assisted mixing step; for example, first a solution of the dried polyurethane in a good solvent for the polymer like **THF** is made, followed by mixing with dried particles or with particles pre-dispersed in a liquid, preferably the same good solvent. In embodiments, a biocompatible dispersion agent may optionally be added to aid in homogeneously dispersing the particles. Depending on the concentration of polyurethane and the amount of particles in the mixture a liquid dispersion or paste-like mixture may be obtained. In a subsequent step the solvent may be removed by known methods like evaporation, preferably at increased temperature and/or reduced pressure. The obtained solidified polymer composition can then be made into a form suitable for use in a shaping step like compression or injection molding, for example by cutting or grinding. Advantages of such solvent-assisted mixing methods include relatively low shear forces and low temperature, that is a low risk of polymer degradation, and a relatively small scale at which it can be operated.

In further embodiments, the method of making polymer composition comprises melt mixing of the components, also called compounding, at a temperature above the softening or melting point of the polyurethane; using known equipment such as a batch-type mixer or a continuous mixer like a single- or twin-screw extruder. Optionally, a biocompatible wetting or dispersion agent may be added before or during melt mixing to enhance dispersion of particles. Before melt mixing, polyurethane and inorganic particles including zirconia are thoroughly dried to minimize hydrolytic degradation in the melt as indicated above. For similar reasons, the melt mixing equipment and mixing conditions are chosen such that the temperature of the composition is kept as low as possible. In embodiments, the polymer composition is made by mixing the dried components on a twin-screw extruder, applying conditions, like screw speed, throughput rate and temperature settings, which result in enough shear or torque for proper dispersion of the particles in polyurethane while minimizing over-heating and polymer chain scission and/or reduction of molar mass. In embodiments, temperature setting of the barrels of the twin-screw extruder is at most 210 °C, preferably at about 205 or 200 °C. It was observed that melt mixing polyurethane with the dried inorganic particles comprising zirconia under such conditions resulted in significantly less polymer degradation than found for the same thermoplastic polyurethane with other mineral fillers like hydroxyapatite and bismuth oxide.

In embodiments, the TPU in the polymer composition from which a bone anchoring part of an implant can be made, or from which an implant part has been made, has a weight-averaged molecular weight Mw of at least 70 kDa. Molar mass and molar mass distribution are typically measured with a GPC method as described in the experimental part. Note that in present disclosure the ISO term 'molar mass' is generally used rather than the still commonly used term 'molecular weight'. It is found that a part made from the polymer composition, which was made by (melt) mixing the TPU with zirconia particles, shows certain minimum desired properties, like a certain elongation at break, if the TPU in the composition has such minimum molar mass. In preferred embodiments, the TPU in the composition has a molar mass Mw of at least 75, 80, 85, 90, 95 or 100 kDa. The molar mass Mw of the TPU that is used for making the composition also meets such minimum values, but is typically higher; yet preferably not so high that its melt viscosity would hamper processing and mixing with zirconia; which also limits the molar mass of TPU in the polymer composition as obtained. In embodiments, the TPU in the polymer composition made has a molar mass Mw of at most 400, 300, 250, or 200 kDa, to result in a balanced combination of processability and mechanical properties.

In embodiments, the polymer composition has an E-modulus of 800-3000 MPa, preferably E-modulus is at least 850 or 900 MPa, and at most 2500, 2000, 1800 or 1600 MPa; as measured on dry-as-molded samples at 20 °C. Alternatively, the polymer composition is characterized by having an E-modulus of 200-700; as measured on wet conditioned samples at 37 °C to mimic biological conditions of its targeted use in an implant. In embodiments, such wet conditioned E-modulus is at least 225 MPa, and at most 600, 550, 500 or 450 MPa. Without wishing to be bound to any theory, the inventors reason that the bone anchoring part of the implant should mimic the mechanical properties, especially the stiffness or modulus, of the surrounding tissue for a more durable result. In this respect the inventors noted that a bone anchoring part of the implant will be mainly in contact with cancellous bone (also called trabecular or spongy bone) rather than with subchondral bone or cortical bone, which form a thin hard layer underneath cartilage and a hard outer layer of a bone, respectively. For example, if the anchoring part has a modulus below said levels, micromotions between the implant and surrounding bone may induce formation of a fibrous tissue interface, instead of desired direct bonding. If the implant has too low stiffness, it may also deform or be damaged upon being loaded. If the implant part has too high a modulus, stress-shielding may occur; in which case a load on the implant, e.g. on a cartilage replacing cap, may be predominately propagated within the implant itself, without loading the surrounding bone tissue. When bone is not loaded, it may remodel and/or be resorbed in areas without loading; ultimately resulting in loosening of the implant. In addition, the present polymer composition shows reduced creep and plastic deformation, compared to materials like unfilled polyurethanes; which contributes to better stability of the implant.

In embodiments, and analogously to the above paragraph, the polymer composition shows an elongation at break during tensile testing (Eab; dry/20 °C) of at least 5%, preferably of at least 10, 20, 30, 40 or 50%, or an Eab (wet/37 °C) of at least 10, 20, 30, 40 or 50%. In embodiments, the polymer composition has a tensile strength at break (TS; dry/20 °C) of at least 30, 35 or 40 MPa or of at least 15, 20 or 25 MPa after conditioning (wet/37 °C). Such stiffness and strength properties of the composition, which are in conditioned state of the same order of magnitude as those of cancellous bone, is found to enhance compatibility and bonding of an implant made from the composition with such living tissue over time. It also eases or improves inserting the implant in a bone hole relative to an implant made from metal, with lower risk of damaging tissue. In addition, an implant made from the polymer composition is found to be sufficiently strong to withstand a typical implantation procedure as may be applied by an orthopedic surgeon; that is to insert the implant into to a hole drilled in the bone at the defected cartilage site, such as by hand using a hammer and a directional guide that may also limit penetration depth, and typically responsive to his sensory perceptions (like noticing changes in sound during hammering).

In embodiments, the polymer composition has a Shore hardness of 76 to 85 ShD, typically of 78-82 ShD (dry/20 °C).In embodiments, the orthopedic implant has a bone anchoring part that substantially consists of the polymer composition comprising a biostable thermoplastic polyurethane and 15-70 mass% of inorganic particles. Such part can be made from the polymer composition by for example an injection molding process. The bone anchoring part may have a smooth outer surface or a textured outer surface, and may optionally have been provided with a surface coating; to for example influence interactions with body tissue.

In embodiments, the outer surface of the bone anchoring part of the implant has a textured surface, such as a surface having a surface roughness Ra of at least 1 µm in order to enhance interaction with surrounding bone tissue after implantation. Such surface roughness may have been introduced during or after making the part; for example by applying a mold that has a certain surface texture; for example as defined by the VDI3400 scale, which is generally used in industry. For an example of a polymeric implant with such textured surface and a method of making it reference is hereby made to WO2019/068903A1. In further embodiments, the bone anchoring part of the implant has a surface roughness Ra of at least 2, 3, 4, 5, 6, 8, 10, 12, 14, 16, 18 or 20 µm and of at most 25 µm.

In embodiments, the orthopedic implant has a bone anchoring part that comprises the polymer composition comprising a biostable thermoplastic polyurethane and 15-70 mass% of inorganic particles, and optionally other components like functional particles or a functional coating on its (smooth or textured) surface; to induce for example osteoconductivity. In some embodiments, the bone anchoring part has a bioactive coating to further promote interactions with tissue, preferably an osteoconductive coating. Various bioactive or osteoconductive coatings, based on both organic and inorganic bioactive material and as described in the art, may be present on the bone anchoring part of present implant.

In further embodiments, bioactive ceramic particles are present at the, optionally textured, surface of the bone anchoring part, which particles for example provide osteoconductive properties to said part of the implant. Suitable bioactive ceramic particles include all inorganic materials that show the capability of direct bonding to living bone, for example by formation of biologically active bone-like apatite through interaction or chemical reaction of the particle surface with surrounding body fluid. Examples of suitable osteoconductive materials include various calcium phosphates, so-called bioglasses and other silica-based ceramics (silicated ceramics). Various types of calcium phosphates have been described for such application, like dicalcium phosphate anhydrate (CaHPO₄; DCPA), dicalcium phosphate dihydrate (CaHPO₄.2H₂O; DCPD), octacalcium phosphate (Ca₈(HPO₄)₂.5H₂0; OCP), tricalcium phosphate (Ca₃(PO₄)₂; TCP), and hydroxyapatite (Ca₁₀(PO₄)₆(OH)₂; HA). Also blends of different types may be applied, or even show advantages; like mixtures of HA and TCP or of HA and bioglass. The ceramic particles may in addition to their main constituents comprise small or trace amounts of other (inorganic) elements or ions, like Si, Na, Mg, Fe, Zn, Ti, Ag, Cu or -SO₄, or -CO₃, which may improve specific properties of the particles.

The term bioglass, including commercial Bioglass^{®} products, refers to mixed inorganic oxides or silicate ceramics that have a surface-reactive glass film compatible with tissues; and may be used as a surface coating in medical and dental implants. The Bioglass^{®} 45S5 grade, for example, is indicated to be a glass composed of 45 mass% SiO₂, 24.5 mass% CaO, 24.5 mass% Na₂O, and 6.0 mass% P₂O₅. The high ratio of calcium to phosphorus in this material would promote formation of apatite crystals; calcium and silica ions can act as crystallization nuclei. Glasses are non-crystalline amorphous solids that are commonly composed of silica-based materials with minor amounts of other inorganic elements. An overview of suitable bioactive ceramics and methods to apply these as coating material to a substrate is for example provided by G. Brunello et al.; DOI: 10.3390/ma12182929.

In an embodiment, the bioactive ceramic particles have a particle size in the range 0.1 - 10 µm. Particle size and size distribution can be measured with SEM or optical microscopy, or with (laser) light diffraction techniques. Within present disclosure the D₅₀. value, e.g. as measured with light diffraction according to ISO 13320:2009 with a Malvern Mastersizer 2000, is defined as the particle size of the bioceramic particles. The particle size does not appear to be specifically critical, but larger particles may be more effective in interacting with body fluid and cells. In view of handleability, ceramic particles having size of at least 0.2 µm, or at least 0.3, 0.4, or 0.5 µm are preferred. In further embodiments, the implant has ceramic particles having size of at most 10, 8, 6, 5, 4, 3, 2 µm, or at most 1 µm at the surface of the bone anchoring part.

The orthopedic implant comprising a bone anchoring part may be a bone anchor, like a plug or screw, and can be used to fixate a further part to bone, like a suture, an artificial ligament or tendon, a meniscus, an acetabular labrum replacement device, as well as a cartilage replacing part. In embodiments, such further part forms an integral part of the implant. In embodiments, the implant comprises at least two parts, that is it further comprises a cartilage replacing part. The cartilage replacing part is typically durably connected to the bone anchoring and at least partly made from a resilient and abrasion resistant biocompatible material. In embodiments, the cartilage replacing part is made from a biostable, biocompatible polymer that has mechanical properties like modulus and hardness that are compatible and comparable with the properties of the natural cartilage layer that it replaces. Various so-called hydrogels have been proposed for cartilage replacing, for example hydrogels based on water-swellable or water-soluble hydrophilic polymers that may be biostable or biodegradable, as for example described in US2011/0218647A1. Typically, a hydrogel is a cross-linked, non-thermoplastic material.

In embodiments of the invention, the implant has a cartilage replacing part that is made from a biostable, resilient thermoplastic polymer, such as a segmented blockcopolymer with hard segments based on a polyester, a polyamide or a polyurethane. In embodiments, the cartilage replacing part is made from a biostable, resilient thermoplastic polyurethane (TPU). An advantage of applying a TPU material for both the bone anchoring part and the cartilage replacing part of the implant is that the two parts may be injection molded to form an article having two adherent or integrated parts; for example by applying an insert molding method or by a multi- or 2-component molding method. Alternatively, the parts may have been welded together using for example a laser source, or joined together using a biostable and biocompatible adhesion promoting composition, including a polyurethane-based composition.

In embodiments, the cartilage replacing part of the implant is made from a resilient biostable, thermoplastic polyurethane having Shore hardness of 55-100 ShA. The polyurethane is typically a block copolymer comprising multiple blocks (also called segments) of polymers (including oligomers) that are chemically distinct, and which show different thermal and mechanical properties, and different solubilities. Often the blocks in a block copolymer comprising two (or more) types of blocks are referred to as being 'hard' and 'soft' polymer blocks, such (chemically) different blocks resulting in microphase separation of hard and soft blocks. The hard block in a block copolymer typically comprises a rigid or high modulus polymer, with a melting temperature (Tₘ) or a glass transition temperature (T_{g}) higher than the use temperature, typically of about 35-40 °C. The soft blocks in the block copolymer generally comprise a flexible, low modulus, amorphous polymer with a T_{g} lower than 25 °C, preferably lower than 0 °C. As for most mechanical properties, thermal parameters like Tₘ and T_{g} are generally determined on dry samples; using well-known techniques like DSC or DMA. In such phase-separated block copolymers, the hard segments function as physical crosslinks for the flexible soft segments, resulting in materials having properties ranging from fairly stiff and rigid to flexible and elastic, depending on the ratio of hard to soft blocks. Depending on type and content of hard blocks, the polyurethane block copolymer may show good stability and elasticity over a desired temperature range without the need for chemical crosslinking; and can generally be processed as a thermoplastic. Thermoplastic polyurethanes are basically a family of polymers with a backbone comprising the reaction product of at least three principle components; which are a diisocyanate, a diol chain extender and a polymer diol or macroglycol. Optionally, a monofunctional compound may be used as a further component functioning as a chain stopper and forming (non-reactive) endgroups. In embodiments, the backbone of the TPU applied in the cartilage replacing part is substantially linear.

In embodiments, the TPU of the cartilage replacing part comprises hard and soft blocks, and optionally end groups, that are (chemically) similar to such blocks and end groups contained in the TPU present in the polymer composition of the bone anchoring part as defined hereinabove, but typically at least with a different ratio hard to soft blocks and/or having different block lengths. Such similar TPUs may enhance interfacial strength between the parts of the implant.

In embodiments, the TPU comprises hard blocks that include urethane groups and optionally urea groups in repeating units, which groups have resulted from reaction of a diisocyanate with a diol and optionally a diamine as respective chain extenders.

Suitable diisocyanates include aromatic, aliphatic and cycloaliphatic compounds, having an average of 1.9-2.1 isocyanate groups per molecule. In an embodiment, the diisocyanate comprises 4,4'-diphenylmethane diisocyanate (MDI), 2,4-toluene diisocyanate, 2,6-toluene diisocyanate (TDI), 1,4-phenylene diisocyanate, hexamethylene diisocyanate (HDI), tetramethylene-1,4-diisocyanate, cyclohexane-1,4-diisocyanate, dicyclohexylmethane-4,4'-diisocyanate (CHMDI), isophorone diisocyanate (IPDI), or a mixture thereof. In an embodiment, the diisocyanate comprises hexamethylene diisocyanate, dicyclohexylmethane 4,4'-diisocyanate, isophorone diisocyanate, or a mixture thereof. In an embodiment, the diisocyanate consists of hexamethylene diisocyanate, dicyclohexylmethane 4,4'-diisocyanate, isophorone diisocyanate, or a mixture thereof. In another embodiment, the diisocyanate comprises 4,4'-diphenylmethane diisocyanate, 2,4-toluene diisocyanate, 2,6-toluene diisocyanate, or 1,4-phenylene diisocyanate. In an embodiment, the diisocyanate consists of 4,4'-diphenylmethane diisocyanate, 2,4-toluene diisocyanate, 2,6-toluene diisocyanate, 1,4-phenylene diisocyanate, or a mixture thereof. In an embodiment, the molar mass of the diisocyanate is from 100 to 500 g/mol. In an embodiment, the molar mass of the diisocyanate is from 150 to 260 g/mol.

Chain extenders are typically low molar mass aliphatic compounds, having two or more, preferably two, hydroxyl or amine groups. Bifunctional chain extenders result in linear, generally thermoplastic polymers, whereas multifunctional chain extenders and/or isocyanates would lead to branched or cross-linked products. In an embodiment, the bifunctional chain extender has a molar mass of at least 60 g/mol, at least 70 g/mol, at least 80 g/mol, at least 90 g/mol, or at least 100 g/mol. In an embodiment, the chain extender has a molar mass of at most 500 g/mol, at most 400 g/mol, at most 300 g/mol, at most 200 g/mol, or at most 150 g/mol. In an embodiment, the chain extender comprises ethylene glycol, diethylene glycol, propylene glycol, dipropylene glycol, 1,3-propanediol, 1,4-butanediol, 1,5-pentanediol, 1,6-hexanediol, or 1,8-octanediol; and/or such corresponding diamines. In embodiments, the thermoplastic polyurethane comprises only diol chain extenders.

In other embodiments, the TPU comprises hard blocks having both urethane and urea linkages. The advantage thereof is enhanced interaction between the hard blocks, resulting in higher softening temperature and/or allowing a higher content of soft blocks; resulting in block copolymers showing enhanced flexibility and elasticity, and excellent flex life or fatigue resistance. Depending on the ratio diol/diamine, the polyurethane chains may show such strong interactions that at a melt processing temperature thermal degradation may occur to such extend that solution processing is to be preferred for optimal performance. Commercially available examples of such relatively low hardness polyurethanes comprising both urethane and urea linkages, also referred to as polyurethane ureas, include Biospan^{®} products (available from e.g. DSM Biomedical BV, Sittard-Geleen NL).

In further embodiments, the thermoplastic polyurethane comprises soft blocks derived from at least one aliphatic polymer diol or polyol, which is chosen from the group consisting of polyethers, polyesters, polyacrylates, polyolefins and polysiloxanes (also called silicones); which polymers are bifunctional with hydroxyl (or amine) terminal groups. Such polymer diols for the soft blocks are understood herein to include oligomers, homopolymers and copolymers, and polyesters are considered to include polycarbonates. Generally known polyurethane block copolymers and methods to prepare these copolymers are described in a.o. US4739013, US4810749, US5133742 and US5229431.

In embodiments of the present disclosure the thermoplastic polyurethane comprises as soft block at least one polymer diol chosen from an aliphatic polyester diol, aliphatic polycarbonate diol, an aliphatic polyether diol, a poly(isobutylene) diol and a polysiloxane diol. Like or chain extenders, also some amine-functional soft blocks can be used, resulting in additional urea linkages. Biocompatibility and biostability of such polyurethane block copolymers in the human body has been proven.

Mechanical and other properties of a thermoplastic polyurethane can be tailored by varying chemical compositions and/or molar masses of the blocks. The hard blocks of a thermoplastic polyurethane contained in the composition of the invention may have a molar mass of about 160 to 10,000 Da, and more preferably of about 200 to 2000 Da. The molar mass of the soft segments may be typically about 200 to 100,000 Da, and preferably at least about 400, 600, 800 or 1000 Da and at most about 10,000, 7500, 5000, 4000, 3000 or 2500 Da. Within the context of present disclosure, molar mass of polymers and oligomers forming blocks in the polymer refers to the number average molar mass (Mₙ), as for example derived from GPC measurements. The ratio of soft to hard blocks can be chosen to result in certain stiffness or hardness of the polyurethane. The hardness of the polyurethane as measured with the Shore durometer hardness test may be from 55 ShA up to 100 ShA, generally representing a tensile modulus range of about 10 to 100 MPa (dry-as-molded). In embodiments, the thermoplastic polyurethane has a hardness of at least 60, 65, or 70 ShA. The advantage of using a TPU of relatively low hardness may be a better cushioning effect in a joint. A higher hardness may result in more durable wear resistance. In other embodiments, the TPU in the cartilage replacing part has a hardness of at most 95, 90 or 85 ShA, for a good balance between resilience and wear resistance. In other embodiments, the TPU in the cartilage replacing part has a tensile modulus (wet conditioned at 37 °C) of about 2-50 MPa, preferably of 5-40 or 8-30 MPa.

In further embodiments of present invention, the thermoplastic polyurethane comprises an aliphatic polyether, an aliphatic polyester, an aliphatic polycarbonate or a combination thereof as soft block, more specifically an aliphatic polycarbonate. Compositions of soft blocks are preferably such that an essentially amorphous oligomer or polymer having a T_{g} below 10, 0, and preferably below -10 °C results. Suitable aliphatic polyethers include poly(propylene oxide) diols, poly(tetramethylene oxide) diols, and their copolymers. Suitable aliphatic polyesters are generally made from at least one aliphatic dicarboxylic acid and at least one aliphatic diol. Aliphatic polycarbonate diols are based on similar aliphatic diols as used for polyester diols, and can be synthesized via different routes as known in the art. Suitable examples include poly(hexamethylene carbonate) diols and poly(tetrahydrofuran carbonate) diols. Such polycarbonate-based TPUs show favorable biocompatibility like hemocompatibility, and enhanced biostability. In an embodiment, the soft block of the TPU is based on a poly(hexamethylene carbonate) diol, a poly(polytetrahydrofuran carbonate) diol, or a mixture thereof. In a preferred embodiment, the TPU comprises a poly(hexamethylene carbonate) diol as soft block.

In further embodiments, the soft block of the TPU comprises a polysiloxane diol such as a poly(dimethyl siloxane) diol, a polycarbonate diol, or a poly(tetramethylene oxide) diol. In an embodiment, the soft block is based on a polysiloxane diol, a polycarbonate diol, a poly(tetramethylene oxide) diol, or a mixture thereof. In an embodiment, the soft block comprises a mixture of two or more of a polysiloxane diol, a polycarbonate diol, or a poly(tetramethylene oxide) diol. Such mixtures allow making biocompatible polyurethanes that show enhanced hydrolytic stability combined with high toughness. In an embodiment, the soft block is based on a mixture of two or more of a polysiloxane diol, a polycarbonate diol, or a poly(tetramethylene oxide) diol. In an embodiment, the soft block comprises a polysiloxane diol and one or more of a polycarbonate diol and a poly(tetramethylene oxide) diol. In an embodiment, the soft block is based on a polysiloxane diol and one or more of a polycarbonate diol and a poly(tetramethylene oxide) diol.

In an embodiment, the soft blocks may further comprise a C₂-C₁₆ fluoroalkyl diol or C₂-C₁₆ fluoroalkyl ether diol. In an embodiment, the soft block in the polyurethane backbone comprises the residue of 1H,1H,4H,4H-Perfluoro-1,4-butanediol, 1H,1H,5H,5H-Perfluoro-1,5-pentanediol, 1H,1H,6H,6H-perfluoro-1,6-hexanediol, 1H,1H,8H,8H-Perfluoro-1,8-octanediol, 1H,1H,9H,9H-Perfluoro-1,9-nonanediol, 1H, 1H, 10H, 10H-Perfluoro-1, 10-decanediol, 1H, 1H, 12H, 12H-Perfluoro-1, 12-dodecanediol, 1H,1H,8H,8H-Perfluoro-3,6-dioxaoctan-1,8-diol, 1H, 1H, 11H, 11H-Perfluoro-3,6,9-trioxaundecan-1, 11-diol, fluorinated triethylene glycol, or fluorinated tetraethylene glycol.

In an embodiment, the C₂-C₁₆ fluoroalkyl diol or C₂-C₁₆ fluoroalkyl ether diol has an Mₙ of at least 150 g/mol, at least 250 g/mol, or at least 500 g/mol. In an embodiment, the fluoroalkyl diol or fluoroalkyl ether diol has a molar mass of at most 1500 g/mol, at most 1000 g/mol, or at most 850 g/mol. In an embodiment, the C₂-C₁₆ fluoroalkyl diol or C₂-C₁₆ fluoroalkyl ether diol is present in an amount of at least 1 mass%, at least 2 mass%, or at least 5 mass%, based on the total mass of the polyurethane. In an embodiment, the C₂-C₁₆ fluoroalkyl diol or C₂-C₁₆ fluoroalkyl ether diol is present in an amount of at most 15 mass%, at most 10 mass%, or at most 8 mass%, based on the total mass of the polyurethane.

In embodiments, the polyurethane may comprise one or more hydrophobic or hydrophilic endgroups. An endgroup is a generally a non-reactive moiety present at a terminal end of a molecule. In an embodiment, the polyurethane comprises an endgroup at each terminus of the backbone; i.e. it has an average of about 2 endgroups. In an embodiment, the endgroup is a linear compound. In another embodiment, the endgroup is branched. An endgroup may have been formed by reacting a terminal isocyanate group present during or after forming the polymer backbone with a co-reactive group on a monofunctional compound, also called chain stopper. For instance, a formulation for forming a polyurethane may comprise a diisocyanate, a polymeric aliphatic diol, a chain extender, and a monofunctional alcohol or amine; like 1-octanol or octylamine to form a C₈ alkyl endgroup.

In embodiments, the endgroup is a hydrophobic endgroup, for example comprising a C₂-C₂₀ alkyl, a C₂-C₁₆ fluoroalkyl, a C₂-C₁₆ fluoroalkyl ether, a hydrophobic poly(alkylene oxide) or a polysiloxane, including copolymers thereof. In an embodiment, the hydrophobic poly(alkylene oxide) is poly(propylene oxide), poly(tetramethylene oxide) or a copolymer thereof. In an embodiment, the hydrophobic endgroup is a polysiloxane, like a poly(dimethyl siloxane). In an embodiment, the endgroup comprises C₂-C₂₀ alkyl, C₂-C₁₆ fluoroalkyl, C₂-C₁₆ fluoroalkyl ether, or a hydrophobic poly(alkylene oxide). Such endgroups may be formed with monofunctional alcohols, including carbinols, or amines of the foregoing. Such polyurethane elastomers having hydrophobic endgroups are found to positively affect properties of the polyurethane and its interaction with other materials, including other polymers like polyolefins and bodily tissue and fluid like blood.

In an embodiment, the hydrophobic endgroup comprises C₂-C₁₆ fluoroalkyl or C₂-C₁₆ fluoroalkyl ether. Such endgroups may be formed with monofunctional alcohols or amines comprising C₂-C₁₆ fluoroalkyl or C₂-C₁₆ fluoroalkyl ether. In an embodiment, the endgroup is formed from 1H, 1H-Perfluoro-3,6-dioxaheptan-1-ol, 1H, 1H-Nonafluoro-1-pentanol, 1H,1H-Perfluoro-1-hexyl alcohol, 1H,1H-Perfluoro-3,6,9-trioxadecan-1-ol, 1H,1H-Perfluoro-1-heptyl alcohol, 1H,1H-Perfluoro-3,6-dioxadecan-1-ol, 1H,1H-Perfluoro-1-octyl alcohol, 1H, 1H-Perfluoro-1-nonyl alcohol, 1H,1H-Perfluoro-3,6,9-trioxatridecan-1-ol, 1H, 1H-Perfluoro-1-decyl alcohol, 1H, 1H-Perfluoro-1-undecyl alcohol, 1H, 1H-Perfluoro-1-lauryl alcohol, 1H, 1H-Perfluoro-1-myristyl alcohol, or 1H,1H-Perfluoro-1-palmityl alcohol.

In another embodiment, the endgroup is a hydrophilic endgroup, that is formed from a hydrophilic monofunctional alcohol or amine compound. Such compound is typically soluble in water and may show surface activity; like a polyethylene oxide or a sulfonate-functional compound. Such hydrophilic endgroups may positively affect interactions with other materials, including biological tissue or fluid.

In another embodiment, the polyurethane comprises a mixture of hydrophobic and hydrophilic endgroups. Such modification allows to control the hydrophobic vs hydrophilic balance of the polymer. A general advantage of using TPUs having endgroups is to modify and control properties of the polymer without incorporating additives that may introduce potential problems with migration from the polymer and implant.

In an embodiment, the endgroup is monomeric and has a molar mass of 200 g/mol or more, 300 g/mol or more, or 500 g/mol or more; and of 1,000 g/mol or less or 800 g/mol or less. In an embodiment, the endgroup is polymeric and has a molar mass of 10,000 g/mol or less, 8,000 g/mol or less, 6,000 g/mol or less, or 4,000 g/mol or less. In an embodiment, the endgroup is polymeric and has a molar mass of 500 g/mol or more, 1,000 g/mol or more, or 2,000 g/mol or more.

In an embodiment, the endgroup is present in an amount of at least 0.1 mass%, at least 0.2 mass%, at least 0.3 mass%, or at least 0.5 mass%, based on the total mass of the polyurethane. In an embodiment, the endgroup is present in an amount of at most 3 mass%, at most 2 mass% or at most 1 mass%, based on the total mass of the polyurethane. In an embodiment, the endgroup is present in an amount of at least 0.1 mass%, at least 0.2 mass%, at least 0.3 mass%, or at least 0.5 mass%; and in an amount of at most 3 mass%, at most 2 mass% or at most 1 mass%, based on the total mass of the polyurethane.

In an embodiment, the polyurethane comprises less than 0.1 mass% of endgroups, based on the total weight of the polyurethane. In an embodiment, the polyurethane is substantially devoid of endgroups. In an embodiment, the polyurethane is devoid of endgroups.

The hard blocks in the TPU are typically based on an aromatic diisocyanate like toluene diisocyanate (TDI) or methylenediphenyl diisocyanate (MDI), and a low molar mass aliphatic diol like 1,4-butanediol. Polyether and polycarbonate polyurethanes may be suitably used for biomedical applications, in view of their flexibility, strength, biostability, biocompatibility and wear resistance. A TPU containing a combination of a polyether and a polysiloxane or a polycarbonate and a polysiloxane in the soft blocks shows a unique combination of properties and may advantageously be used as the polyurethane in the polymer composition. Commercially available examples of such polymers include Pursil^{®} and Carbosil^{®} products (available from DSM Biomedical BV, Sittard-Geleen NL).

In a further embodiment, the TPU may be a blend of two or more polymers, for example a blend of two biocompatible TPU grades having different hardness, like a combination of 50 ShA and 50 ShD grades; or be a blend of two TPU grades differing in soft blocks and/or in endgroups. The TPU can also be a blend of a TPU with another biocompatible polymer, preferably another low modulus, elastomeric material. Typically, such other biocompatible polymer is dispersed within the TPU and forms at most 40 vol% of the blend, preferably at most 30, 20 or 10 vol%.

In other embodiments the TPU may comprise one or more customary additives that are allowed for the targeted use of the TPU in an implant; in addition to e.g. catalyst residues. Examples of additives include stabilizers, anti-oxidants, processing aids, lubricants, surfactants, antistatic agents, biologically active compounds like anti-inflammatory agents, filler particles including e.g. such inorganic particles as contained in the polymer composition of the bone anchoring part, etc.. The additives may be present in the typically effective amounts as known in the art, such as 0.01-10 mass% based on the amount of the polyurethane, preferably at most 8, 6,4, 3, or 2 mass%. In another embodiment, the TPU is substantially free of additives.

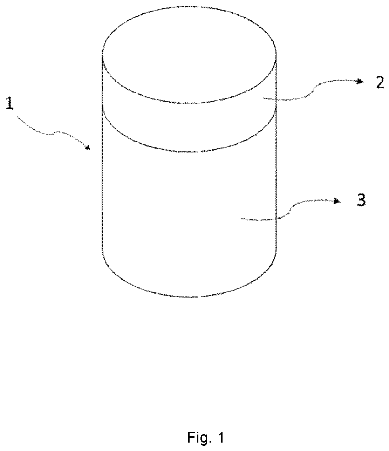

The cartilage replacing part of the implant generally has a thickness similar to or substantially matching that of the cartilage layer in the joint for which it will be a local replacement. Such a thickness is aimed at that after implanting, the sides of the cartilage replacing part substantially only contact adjacent original cartilage; whereas the top surface of the part is available for abutting the opposite cartilage surface in the joint. It is found that such design results in good integration with the natural cartilage and reduces risk of being damaged by contacting harder bone tissue. Similarly, the implant is designed such that contact of the more rigid bone anchoring part with the natural cartilage is limited or even prevented to reduce corresponding risk of damaging. This is further illustrated in Figures 1-3, see below.

In embodiments, the cartilage replacing part of the implant has a thickness of about 0.2-5 mm, preferably of at least 0.4, 0.6, 0.8, 0.9, 1.0, 1.1, 1.2, 1.3, 1.4 or 1.5 mm, and of at most 4.5, 4.0, 3.8, 3.6, 3.4, 3.2, 3.0, 2.9 or 2.8 mm. In embodiments, the cartilage replacing part of the orthopedic implant for cartilage replacement is a layer of substantially constant or uniform thickness, such that its properties and performance in the joint are similar over its surface area.

In embodiments, the implant having for example a mushroom-like design with cap and stem parts, the cap, and thus the cartilage replacing part or layer, has a curved or contoured top surface; like the surface of the joint wherein it will be placed. Such curvature may be characterized by one radius, or by two or more different radii in different directions; like a biplanar curvature. The curvature is typically convex, but may also be a combination of convex and concave, like in case of a saddle-like surface of a joint. A cartilage replacing part or layer with multiple contour radii may have a thickness that varies somewhat within said thickness ranges, preferably thickness of the layer is substantially uniform. In case the cartilage replacing part of the orthopedic implant is of substantially uniform thickness, the top surface of the bone anchoring has a similar curvature as described above for the cartilage replacing part.

The curvature of bones in joints varies widely and from person to person, a.o. depending on the joint, location therein, gender and age; in the knee joint for example curvatures may be characterized by radii of 15-70 mm. Considering that defects most frequently occur in in the center part of a joint, embodiments of the invention include implants that have at least one surface curvature with a radius in the range 20-60 mm, as such implants may be suitable for use with most patients.

The orthopedic implant comprising a bone anchoring part and optionally a cartilage replacing part may have various forms or shapes; preferably the implant, or at least part thereof like the bone anchoring part, has axial symmetry.

In an embodiment, as schematically shown by the simplified drawing in Figure 1 representing an angled top-view, the implant **1** is of cylindrical shape with a substantially constant diameter, wherein a larger section **3** of the cylinder represents the bone anchoring part and the cartilage replacing part is a section (or layer) **2** at one end of the cylinder. The diameter of the cylinder may be 5-20 mm, 10-18, 12-17 or typically about 15 mm. Alternatively, the substantially cylindrical form may show some tapering from top to bottom, with the cartilage replacing part or layer having a diameter of at most 10% larger than the smallest diameter of the bone anchoring part. A slightly tapered (bone anchoring) part is found to be advantageous in releasing the part from a mold wherein it is made, but also in placing in a bone hole and in securing contact with bone upon implantation. Relative to the lengthwise orientation of the anchoring part, the outside may show a tapering of 1-5°, preferably of at least 1.5, or 2.0° and at most 4.5, 4.0, 3.5 or 3.0°.

Although not indicated in Fig. 1, the edges of the cylinder may be somewhat rounded to reduce stress concentrations when loaded, and section 2 may be curved. The cartilage replacing part, or at least the top surface thereof, may be substantially flat, but is preferably curved or contoured to mimic the curvature of the articulating joint wherein it is to be implanted. In embodiments, the top curvature is defined by at least two different radii, like by two radii in rectangular directions to each other; i.e. the surface has a biplanar curvature. In this way the typical curvature of the articulating surface of a bone in a joint may be simulated in a relatively simple manner. This not only simplifies production of molds and implants, but also reduces the number of implant plugs having different sizes and/or geometries, i.e. plugs with different curvatures and optionally different diameters, which should be kept in stock or are present in a kit to allow selecting the most suitable implant for a given patient.

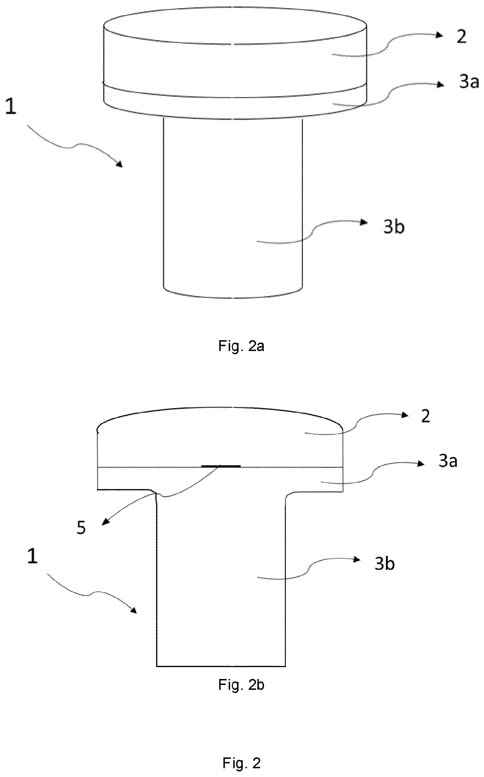

In other embodiments, the implant **1** has a mushroom-like shape with a cap and at least one stem, with the cap being wider, e.g. having a larger diameter than the stem. Such implant may have a basic axial symmetry with a round cap; but the cap may also be oval or elliptical and/or the implant may have more than one stem. In embodiments, the implant has substantial axial symmetry in the at least one stem of the bone anchoring part. The simplified drawings as shown in Figures 2A and 2B, schematically represent an angled top-view and a side view of such mushroom-like implant. In these drawings, the stem **3b** is cylindrical with a substantially constant diameter, but may be alternatively tapered or conical, and forms together with the lower section **3a** of the cap the bone anchoring part **3.** Relative to its lengthwise orientation, the outside of the stem may show a tapering of 1-5° as described above; wherein the diameter of the stem decreases slightly from cap to bottom end. Both sections **3a** and **3b** that form the bone anchoring part **3** are preferably made from the same polymer composition, like the composition described in this disclosure. The top section (or layer) **2** of the cap represents the cartilage replacing part, which is made from e.g. resilient TPU and which is supported by and adhered to the section **3a** of the bone anchoring part. The diameter of the stem may be about 5-15 mm, typically about 6-10 mm; and the cap may have a diameter of about 5-25, 10-20, 12-18, or about 15 mm. In practice, typically a series of implants of different sizes, especially with different cap sizes, may be made available for example as part of a kit; allowing selection of a suitable implant before or during surgery, depending on the patient that is to be treated. Edges of the plug, and transitions like from cap to stem and from side wall to bottom of a stem are preferably rounded, to reduce stress concentrations upon load during implantation and/or use (not shown in Fig. 2).

In embodiments, the implant has rounded edges and/or transitions, which may have a rounding radius of 0.1-4.0 mm, also depending on other dimensions of the implant. Load distribution simulations on implanted plugs, for example by applying finite element analyses (FEA; also referred to as finite element modelling, FEM), showed that forces applied to the surface of the cartilage replacing part can be better distributed over the implant and high local stress concentrations may be prevented by optimizing dimensioning of said edges and transitions. In embodiments, edges and transitions of the implant are rounded with a radius of at least 0.2, 0.5, 1.0, 1.5, or 2.0 mm and of at most 3.5 or 3.0 mm.

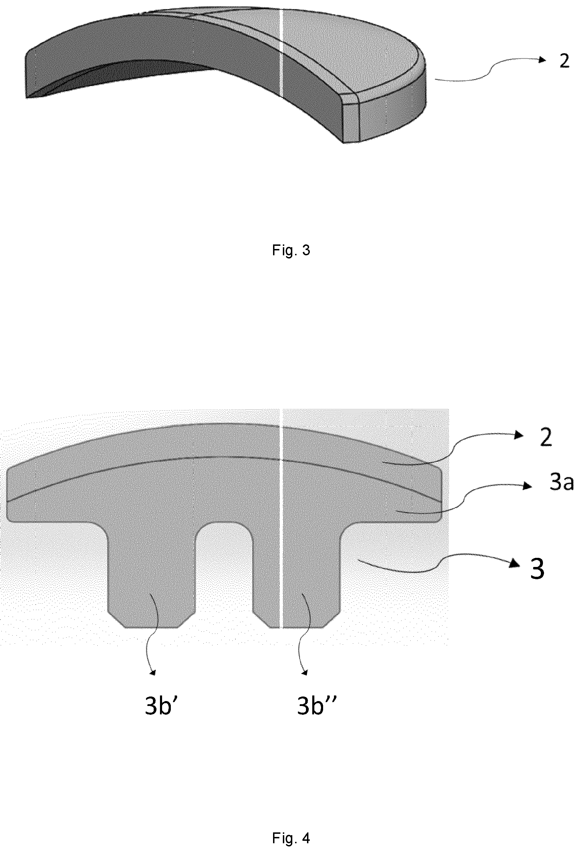

In embodiments and as indicated above, the top surface of the cartilage replacing part 2 of the implant may be curved or contoured in at least one, preferably in at least two directions to mimic curvature of the articulating joint wherein it may be implanted. In embodiments, the cap has a biplanar surface curvature, i.e. the top curvature is characterized by two different radii, in rectangular directions to each other. Such embodiment is further illustrated by Figure 3, showing only a part of a cartilage replacing part **2** of an implant, which has biplanar curvature and is of substantial uniform thickness. Although such implant with biplanar curvature may not fully reproduce the natural curvature, such approximation allows simulating a typical curvature of the articulating surface of bone in a joint in a relatively simple manner. In addition, a biplanar curvature simplifies production of molds and of implants therewith , and limits the number of plugs of different geometries and sizes, like with different curvatures and optionally different diameters of stem and/or cap, which would need to be kept in stock or be present in a kit of parts for selecting the most suitable implant for a given patient.

In other embodiments, the implant has a surface curvature that can be described by one radius. In further embodiments, the implant has surface curvature that can be described by more than two radii; to define a surface that more closely follows a complex surface curvature at a particular location of a joint. In addition, the top surface typically has a convex curvature in one or more directions; but may also have a combination of convex and concave curvatures, like in a saddle-shaped part of a joint surface. In general, the radius or radii of curvature of the cartilage replacing part of the implant of present disclosure may depend on the type of joint and on the patient, and the skilled person will be able to select suitable ranges. In embodiments, the radii of curvature are in the range 15-70 mm, preferably radii are at least 20 or 25 mm and at most 65, 60, 55 or 50 mm.

In embodiments and referring again to Figure 2, at least part of the underside of the cap **3a,** forming a section of the bone anchoring part, is basically flat. This section **3a** supports the cartilage replacing part **2,** including to distribute forces acting upon said layer; connects part **2** and the stem section **3b;** and plays a role in anchoring to bone. Upon implanting, a bone hole will be made of a dimensions the same as (or slightly smaller than) those of the stem **3b** of the plug, whereas damaged cartilage will be removed with such diameter and depth that cancellous bone tissue is exposed and the side and underside of section **3a** will contact the patient's bone tissue; as will the stem **3b.** The cartilage replacing part **2** would then mainly or only contact natural cartilage at its side.

Dimensions of the bone anchoring part 3, like thickness of **3a** and diameter and length of **3b,** will be dependent on different factors; like the joint wherein it will function as implant, typical loadings, and the size of the cartilage replacing part. The skilled person will be able to choose suitable dimensions based on above considerations, for example with the help of some model calculations. In embodiments, section **3a** may have a thickness of about 1-5 mm; preferably at least 1.5 or 2 mm and at most 4.5, 4, 3.5 or 3 mm. In case of a curved cartilage replacing part of substantially constant thickness, the top surface of **3a** of the bone anchoring part **3** will have a similar curvature and its thickness may not be constant but may inverse from edge to center. Thickness may be about 1-7 mm; preferably at least 1.5 or 2 mm and at most 6.5, 6.0, 5.5, 5.0, 4.5, 4, 3.5 or 3 mm; a.o depending on the dimensions (esp. diameter) of the cap part. Optionally, in case of relatively large plugs with a large cap, a connection or transfer zone between 3a and 3b may be rounded with a relatively large radius or be gradual, like stepwise or conical, to better distribute loads on an edge zone of part 2 of the cap. Alternatively, reinforcing ribs connecting 3a and 3b may be used to further support section 3a and further strengthen the implant and its durability.

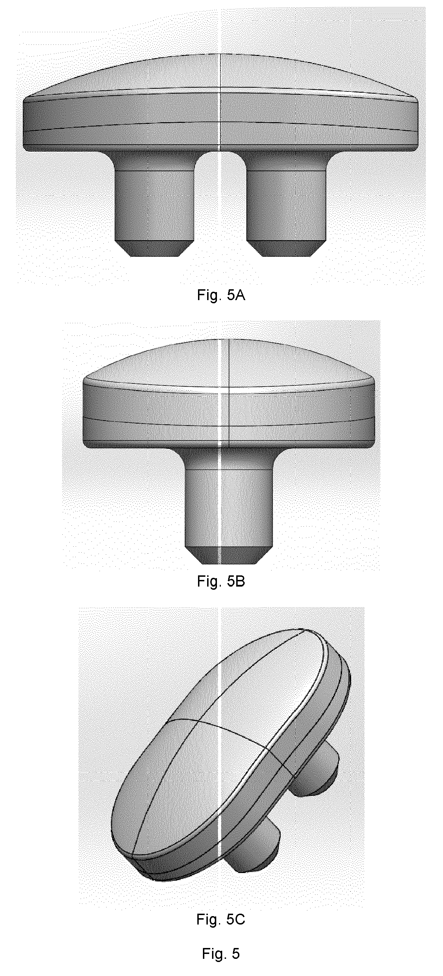

In other embodiments, the orthopedic implant has no axial symmetry as a whole, but can for example be thought to comprise two similar implant parts with axial symmetry that have partly fused together with their caps; like Siamese twins. An example of such implant, which has a mushroom-like shape, having a substantially oval or elliptical cap with a contoured top surface and two substantially identical, cylindrical or tapered stems, is schematically shown in a cross-sectional view in Figure 4. Herein the top layer **2** of the cap, having a substantially uniform thickness, forms the cartilage replacing part and the underside of the cap **3a** with the two stems **3b'** and **3b"** together form the bone anchoring part **3.** Note the rounded edges and transitions from 3a to 3b. In Figure 5 such an implant is further exemplified by different schematic views. Figures 5A and 5B provide side views of the respective longer and shorter side of the non-cylindrical implant; showing the single cap and both or only one of the stems, respectively. Fig. 5C represents an angled top view of the implant, showing the biplanar-contoured surface of the oval cap.

In embodiments, the size of the bone anchoring part **3** of the implants, or sections **3a** and **3b** thereof, as for example shown in Figures 1 and 2, respectively, are selected from a limited set of standard lengths and/or diameters, so as to simplify surgical procedures like preparing a suitable bone hole, and to limit the number of implants to be kept in stock, or to be included in a kit of parts. Typically, such bone anchoring part has a length of about 5-10 mm, for example about 7 mm, and the diameter may be 5-20 mm for cylindrical implants or 5-15 mm for mushroom-like designs.

In embodiments, the orthopedic implant comprises a bone anchoring part and a cartilage replacing part, wherein the bone anchoring part has a surface that contacts the cartilage replacing part, which surface is structured with protrusions and/or recessions having height and/or depth, and width of 0.05-3.0 mm. In embodiments, said dimensions are at least 0.1, 0.2, 0.3, 0.4 or 0.5 mm and at most 2.5, 2.0, 1.5 or 1.0 mm. Width relates to the cross-sectional dimensions of a (semi-) round protruding or recessed structure, and to the smaller dimension in case of an elongated structure (length may be longer, e.g. in case of a bend or spiraling structure or infinite in case of a circular structure). These structures generally have no sharp, but rounded edges; for example with a rounding radius of 0.1-0.5 mm. The bone anchoring part having such surface structure means that the cartilage replacing part connected thereto has a similarly structured surface in a positive-negative relationship. In other words, such implant has a structured interface between the bone anchoring part and the cartilage replacing part.

In another aspect, the invention provides an orthopedic implant, like a biostable implant suitable for use in repair of damaged cartilage tissue, wherein the implant comprises a bone anchoring part and a cartilage replacing part, and wherein a surface of the bone anchoring part that contacts the cartilage replacing part is structured having protrusions and/or recessions with height and/or depth, and width of 0.05-3.0 mm. Width relates to the cross-sectional dimensions of a (semi-) round protruding or recessed structure, and to the smaller cross-sectional dimension in case of an elongated structure (length may be longer, e.g. in case of a bend or spiraling structure or infinite in case of a circular structure). These structures generally have rounded edges; for example with a rounding radius of about 0.2 mm. The bone anchoring part having such surface structure means that the cartilage replacing part connected thereto has a similarly structured surface in a positive-negative relationship. In other words, such implant has a structured interface between the bone contacting part and the cartilage replacing part.

In embodiments of the orthopedic implant having a bone anchoring part with a structured surface, the bone anchoring part comprises a rigid polymer composition, such as a polymer composition comprising a biostable thermoplastic polyurethane (TPU) and 15-70 mass% of inorganic particles comprising zirconia particles, including any of the features as described in the various embodiments herein above, and in any possible combination.

In further embodiments, said orthopedic implant having a bone anchoring part with a structured surface comprises a cartilage replacing part that is made from a resilient, abrasion-resistant, biocompatible material, like from a biostable TPU including any feature as described in the various embodiments herein above, and in any possible combination.

Without wishing to be bound to any theory, inventors believe that a structured interface between the bone contacting part and the cartilage replacing part results in a larger contact surface, and in constrained or reduced deformation of the resilient cartilage replacing layer under changing loads; and therewith in improved adhesion between the parts. In addition, the type, size and number of structures (protrusions and/or recessions) may be selected to affect distribution of loads applied to the surface of the cartilage replacing part to the bone anchoring part and to prevent occurrence of stress concentrations that may induce local defects; for example initiation of delamination at the interface between the parts. The structured interface may have structures one design, but also a combination of different protruding and/or recessed structures may be present. Identification and selection of suitable protruding and/or recessed structures and their effects may be supported by model calculations, using for example FEA tools.

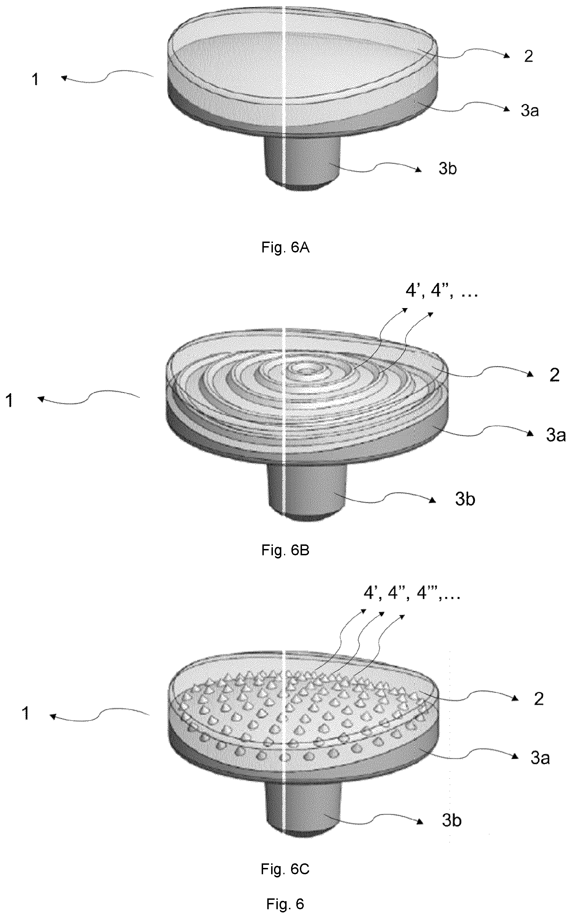

In embodiments, the orthopedic implant has a bone anchoring part with a structured surface having protrusions and/or recessions, wherein the protrusions comprise hills or ridges that extend from and/or valleys that are recessed in the surface, which ridges or valleys may be continuous or discontinuous, and which may more or less follow the outer contour of the implant; like in circles of decreasing diameter; as schematically shown in Figures 6. Figure 6A depicts a mushroom-like implant **1** having a (optionally transparent or translucent) cartilage replacing part **2** contacting the smooth (unstructured) surface of section **3a** of the bone anchoring part **3.** In Figure 6B a number of concentric ridges **4** (4', 4", etc.) are shown to protrude from the surface of **3a** into part **2** (which has corresponding valleys). Another exemplary embodiment is shown in Figure 6C, wherein a multitude of individual cone-like protrusion (4', 4", etc.) are present at the surface of **3a** (with corresponding recessions in part **2).**

In embodiments, the orthopedic implant has a bone anchoring part with a structured surface having protrusions and/or recessions **4,** wherein protruding structures have a height that is most 50% of the thickness of the cartilage replacing part **2** into which they protrude, preferably said height is at most 45, 40, 35, 30 or 25 % of the cartilage replacing layer thickness. Similarly, recessed structures **4** may have a depth that is at most 50, 45, 40, 35, 30 or 25 % of the thickness of section **3a** of the bone anchoring part.

In embodiments, the orthopedic implant has a bone anchoring part with a structured surface having protrusions and/or recessions **4** that have height and/or depth and width of 0.05-3.0 mm. In embodiments, said dimensions are at least 0.10, 0.15, 0.20, 0.25, 0.30, 0.35, 0.40 or 0.45 mm and at most 2.5, 2.0, 1.8, 1.6, 1.4, 1.2 or 1.0 mm. In other embodiments, these structures have no sharp, but rounded edges; for example with a rounding radius of 0.1-0.5 mm.

In another aspect, the invention provides an orthopedic implant, like a biostable implant suitable for use in repair of damaged cartilage tissue, the implant having a bone anchoring part and a cartilage replacing part, wherein the cartilage replacing part comprises a layer of resilient and abrasion resistant biocompatible material, like a biostable thermoplastic polyurethane, the layer having a substantially constant or uniform thickness of about 0.2-5 mm, preferably of at least 0.4, 0.6, 0.8, 0.9, 1.0, 1.1, 1.2, 1.3, 1.4 or 1.5 mm, and of at most 4.5, 4.0, 3.8, 3.6, 3.4, 3.2, 3.0, 2.9 or 2.8 mm, and wherein the cartilage replacing part and the top surface thereof is contoured in two or more directions. In embodiments, such orthopedic implant may have one or more further features corresponding to those as described in the various embodiments in this disclosure, and in any possible combination.

In further embodiments of the implant, the top surface of the cartilage replacing part of the implant is contoured in two or more directions, and as a result the implant such as a plug has no axial symmetry. In such case it is important that the implant can be placed with a proper orientation of said contouring relative to the bone curvature at the targeted implant site. In order to ease or enable implanting with such desired orientation during surgery, the orthopedic implant may further contain an orientation marker, which may be visible with the eye and/or with an imaging technique.

In embodiments, the implant has a cartilage replacing layer that is transparent or translucent, and an orientation marker is present at the interface of bone anchoring and cartilage replacing parts. For example, in case the implant contains a bone anchoring part having a structured surface with protrusions and/or recessions as described above, said protrusions and/or recessions may be arranged such that they also function as a visual orientation marker. Alternatively and in other embodiments, a small elongated part, having optical properties like color distinguishable from the other parts of the implant, is present at said interface, which elongated part may have been inserted between subsequent steps of making said parts, as described hereafter.

In further embodiments, a relatively small, radiopaque, elongated part is present as an orientation marker, like a small length of thin metal wire or a number of small metal pieces in a row. An orientation marker like a metal wire can for example have a length of about 2-6 mm, and may be a tantalum wire. In embodiments, such elongated radiopaque marker is present between the bone anchoring part and the cartilage replacing part, for example with its length oriented in the direction of the larger curvature radius in the top surface of the cartilage replacing part. Such orientation marker is represented as line **5** in Figure 2b. in such case, a surgeon can thus not only place the plug in a desired orientation during operation; but can check also post-operative its position and any potential change therein using a suitable medical imaging technique.

In a further aspect, the invention relates to a method of making said orthopedic implant comprising a bone anchoring part, comprising forming the implant with an injection molding process comprising a step of molding the bone anchoring part from the polymer composition as defined in the above, preferably comprising a biostable thermoplastic polyurethane and 15-70 mass% of zirconia particles. Such methods are well known to a person of average skill in the art.

In other aspects, the invention relates to a method of making said orthopedic implant comprising a bone anchoring part and a cartilage replacing part, the method comprising forming the implant with a multi-component injection molding process comprising a step of injecting the polymer composition as defined herein above, preferably comprising a biostable thermoplastic polyurethane and 15-70 mass% of inorganic particles comprising zirconia, into a mold comprising an insert in the form of the cartilage replacing part to form the bone anchoring part, and subsequent steps of removing the insert from the mold, and of injecting a resilient and abrasion resistant biocompatible material like a TPU into the mold partly filled with the polymer composition to form the cartilage replacing part. Making implants by multi-component molding techniques from different polymer grades is known in the art. For example, WO2011/098473A1 describes making orthopedic implants, like meniscus or spinal disc implants, which have two or more distinct sections that each comprise a different, but chemically related, polymeric material; like different TPU grades.

In a further aspect, the invention relates to a method of making an orthopedic implant comprising a bone anchoring part and a cartilage replacing part, the method comprising forming the implant with a multi-component injection molding process comprising a step of injecting a rigid polymer composition into a mold comprising an insert in the form of the cartilage replacing part and having protrusions and/or recessions of 0.05-3.0 mm in height and/or depth and width at the surface contacting the top of the bone anchoring part to be formed, and subsequent steps of removing the insert from the mold, and of injecting a resilient and abrasion resistant biocompatible material into the mold partly filled with the polymer composition to form the cartilage replacing part of the implant.

In embodiments, the method comprises injecting a rigid polymer composition comprising a biostable thermoplastic polyurethane and 15-70 mass% of inorganic particles comprising zirconia and of injecting a resilient and abrasion resistant biocompatible material, wherein the composition and material are as defined herein above, including any features and combination thereof.

In other embodiments, the various methods of making an orthopedic implant may comprise a further step of inserting an orientation marker like a radiopaque marker to the implant, preferably between the step of injecting the polymer composition to form the bone anchoring part and the step of injecting the biocompatible material into the mold. The radiopaque marker, like a piece of thin tantalum wire, is preferably positioned in line with the direction of one of two or more different radii of curvature of the contoured top surface, to for example allow monitoring orientation and proper positioning of the implant during and after implantation.

In embodiments, the mold used in said methods has been provided at the bone anchoring forming part thereof with a defined surface roughness, for example having a roughness according to VDI3400 36 to result in a molded part having a bone anchoring part with a surface roughness Ra of at least 5 µm; see for example the method described in WO2019/068903A1.

In further embodiments, the method of making said orthopedic implant further comprises a step of providing the surface of the bone anchoring part of the molded implant with bioactive, preferably osteoconductive, particles or a bioactive, preferably osteoconductive coating; for example by treating the -optionally textured-surface with a dispersion of bioactive ceramic particles like calcium phosphates in a solvent for the polyurethane comprised in the polymer composition. Details of such solvent-based surface treatment are described in WO2019/068903A1.

In another aspect, the invention relates to a surgical kit of parts comprising at least one orthopedic implant as described herein above, more specifically to a surgical kit comprising a set of at least two implants having different sizes. Implants having different sizes may for example differ in size and/or curvature of the cartilage replacing part, and/or dimensions of the bone anchoring part. Such surgical kits may further comprise auxiliary tools for preparing the implant location to receive one or more orthopedic implants, like guiding and drilling tools for making a bone hole, as well as tools to insert the orthopedic implant into a bone hole.

Further aspects include the use of an orthopedic implant as described herein above and the use of a surgical kit as described herein above in repairing degenerated or damaged cartilage tissue, like in a knee joint of a mammal. Such use for local treatment of damaged cartilage in joints may delay and possibly even avoid the need for a total joint replacement surgery, like a TKR.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein is merely intended to serve as a shorthand method of referring individually to each separate value falling within the range, and each separate value is incorporated into the specification as if it were individually recited herein. The use of any and all examples, or exemplary language (e.g., "such as" or "like") provided herein, is intended merely to better illustrate the invention and does not pose a limitation on the scope of the invention unless otherwise claimed. No language in the specification should be construed as indicating any non-claimed element as essential to practicing the invention.

Preferred embodiments of the inventions are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the inventions to be practiced otherwise than as specifically described herein. Accordingly, the inventions include all modifications of the subject matter recited in the claims appended hereto.

While certain optional features are described as embodiments of the inventions, the description is meant to encompass and specifically disclose all combinations of these embodiments unless specifically indicated otherwise or physically impossible.

The experiments and samples below further elucidate embodiments of the inventions, but of course, should not be construed as in any way limiting the scope of the claims.

### Experimental part

### Methods

### Molar mass

Molar masses and molar mass distribution of samples were measured by GPC (Gel Permeation Chromatography, also referred to as Size Exclusion Chromatography or SEC) as described in ASTM D5296-11, on a Viscotek GPCMax VE2001 system equipped with a TDA302 triple detector array and 3 Phenogel^{™} columns (10 µm 10E6A, 10 µm 10E4A, and 10 µm 100A). Detectors and columns were operated at 80°C. Polymer samples were dissolved at concentration of 1.0 mg/ml in DMF containing 0.05 mass% LiBr and 300 mg/l of DHT, at 70°C for at most 4 hours and filtered via 0.2 µm PTFE membrane. This solvent composition was also used as eluent. Molar mass calculations were based on a calibration curve, which was obtained with EasyCal polystyrene standards and adjusted for results with a polyurethane sample of known molar mass.

### Hardness

Hardness of molded samples was measured using a Zwick Shore Durometer 3131 following ISO 868 (average of 5 tests, measurement time 15 s, at 20.9 °C / 51.1 %RH).

### Tensile properties

Tensile modulus, (ultimate) tensile strength and elongation at break, and were measured on a Zwick Z010 universal tensile testing apparatus, equipped 2.5 kN pneumatic grips and a temperature chamber, with a method based on ISO 527. Injection molded test bars (ISO 527 type 1BA) were tested dry-as-molded at 20 °C and at 37 °C after conditioning in water of 37 °C,. Samples used for dry-as-molded measurements were dried overnight using an 80 °C vacuum oven with a small N₂ flow and stored in a closed box with silica gel Samples were conditioned by submersing samples in water kept at 37 °C and measuring mass increase every 24 h, until less than 0.1 mass% change (typically at least 360 h). Each sample was stored at said conditions until shortly before placing it in the temperature chamber of the tensile tester. Directly before testing, the width and thickness of the samples were measured at the center of the specimens. The sample was placed in the grips with a grip-to-grip separation of 54 mm at the start position. A pre-load of 0.5 N was applied before starting the tensile experiments. Modulus of a sample was determined within the first 0.05 and 0.25 % of strain, at a speed of 1 mm/min. Afterwards, the stress and strain measurement was done at 50 mm/min until the sample fractured, elongation until 60% strain was measured with an extensometer.

### Particle size

Particle size distribution and particle size (D1₀, D₅₀ and D₉₀) were measured on samples dispersed in water with a Malvern Mastersizer 2000, according to ISO 13320:2009.

### Crystallization behavior

Differential scanning calorimetry (DSC) was performed using a standard heat flux DSC of Mettler Toledo. Samples of about 5 mg mass are cut from pellets, weighed with a precision balance and encapsulated in (crimped) aluminum pans of known mass. An identical empty pan is used as a reference. Nitrogen is purged at a rate of 50 ml/min. Heating-Cooling-Heating cycles were applied for determining the parameters that numerically characterize the thermal behavior of the investigated materials. The temperature program applied was: [1] 0.0-70.0 °C, 10.00 K/min; [2] 70.0 °C, 60.00 min; [3] 70.0-90.0 °C, -10.00 K/min; [4] -90.0 °C, 10.00 min; [5] -90.0-240.0 °C, 10.00 K/min; [6] 240.0 °C, 2.00 min; [7] 240.0-90.0 °C, -10.00 K/min; [8] -90.0 °C, 10.00 min; [9] -90.0-240.0 °C, 10.00 K/min.

### Polymer compositions

### Experiments 1 - 3.

In the experiments a polycarbonate urethane based on MDI, butanediol and poly(hexamethylene carbonate)diol, having a hardness of 75 ShD and a mass average molar mass Mw of 388 kDa was used, after having been dried during 24 h at 80 °C to a moisture content of 113 ppm. Hydroxyapatite of particle size 5 µm (Merck, Hydroxyapatite for Bioceramics) was dried for 24 h at 120 °C to reach a level of about 2650 ppm of moisture.

The polyurethane and filler components were melt-mixed on a Coperion ZSK Mc¹⁸ twin-screw extruder, equipped with 2 Colortronic LabLine feeders and having a die-plate with 1 opening of 3 mm. Polyurethane granules were dosed on the intake barrel and HA powder was dosed on the intake side-feeder to barrel 2, at a mass ratio of TPU/HA of 80/20. Temperature setting of all zones was 190 °C. The screws rotated at 150-200 rpm with a throughput of about 2.5 kg/h, torque level varied somewhat within the range 50-70%. Extrusion conditions were selected to result in a melt temperature of at most about 200°C; to enable stable compounding resulting in a smooth and regular strand. The extruded strand was cooled in a water bath with 2 m total cooling length, and then cut into pellets using a motorized strand pick-up and Scheer 50E pelletizer (operated at slow speed).

The extruder was rinsed before and after a mixing experiment with the polyurethane during 5-10 min at 2 kg/h throughput.

The obtained pellets were injection molded into test bars on an Xplore IM12 micro injection moulder, after drying the pellets under vacuum/N₂ at 120 °C during 24 h. Barrel temperature was set at 200°C and mold temperature at 100 °C. After a melting time of at least 5 min., the material was injection into the vented mold at an injection pressure of 10 bar during 2.2 s and packing pressure 10 bar during 10 s.

Tensile properties were determined on dry-as-molded specimen at room temperature (20 °C) as well as at 37 °C in wet state to mimic conditions when used as implant material.

In Table 1 results are summarized as (comparative) Experiment. 2. Also presented in this table are molar mass data (Mw) as determined on pellets and molded test specimen.

Experiment 1 concerns results of reference tests that were performed on the unfilled polyurethane pellets, which were injection molded and tested analogously to the above Exp. 2, but applying a molding temperature of 235 °C and pressures of 15 bar.

Experiment 3 was performed analogously to Exp. 2, but the amount of HA dosed to the extruder was 40 mass%.

The tensile measurement results as summarized in Table 1 suggest that hydroxyapatite particles act as a non-reinforcing filler in the polyurethane; the dry modulus is not significantly altered, the tensile strength and elongation are markedly decreased, but water up-take is reduced and has less effect on properties (20 °C/dry vs 37 °C/wet). Shore hardness of the molded bars increased from 75.7 ShD (Exp. 1) to 80.2 ShD (for Exp. 2). The GPC results indicate a significant lowering in molar mass when compared to the unfilled material, which is likely caused by residual moisture contained in the crystalline HA particles inducing degradation of the polymer during melt processing steps; predominantly during the compounding step. The observation that during compounding the melt temperature should be kept below 200 °C, because otherwise no stable strand extrusion was possible, would support such explanation.

### Experiments 4.

Analogously to the compounding procedure of experiments 2 and 3, bismuth oxide particles (Bi₂O₃) of particle size 4 µm (D₅₀; 5N Plus Product Data Sheet (Helos/Rodos), dried at 120 °C during 24 h to a moisture content of 153 ppm, were used instead of HA. Various variations in processing conditions were tried, but trials were stopped when no stable processing appeared possible, and/or foaming and discoloration of the extrudate was observed. This likely is caused by excess degradation of the polyurethane. No further experiments were done with this material.

### Experiments 5 - 6.

In these experiments, zirconium oxide TZ-0 (Tosoh Europe BV)), a free-flowing powder with (primary) particle size of 0.04 µm (according to Tosoh brochure), was added as filler to the polyurethane during compounding. Samples were dried at 120 °C during 24 h and compounded with the same 75 ShD polyurethane applying the procedure of experiments 2 and 3. By setting the temperature of the barrels at 200 °C and increasing screw speed to 300 rpm, strand breaking during compounding was prevented and particle dispersion appeared to be improved. Torque level was limited to 60-70%, and melt temperature at most 212 °C. Dosing behavior of the powder was very good, but thick-thin variations as observed in the extruded strand suggest the particles were not well dispersed.

Addition of these zirconia particles at 20 and 40 mass% loading results in a decrease in strength; at 40 mass% loading the composition is even rather brittle. This is likely caused by insufficient dispersion of the agglomerated primary particles in the polyurethane matrix. A pretreatment of the particles and/or addition of a dispersion agent may be applied to enhance dispersibility; and to improve mechanical properties.

### Experiments 7 - 8.

Applying the same conditions and procedure as in Exp. 5-6, compositions of 75 ShD polyurethane with zirconium oxide TZ-Y3-E (Tosoh) were made. This zirconia grade TZ-Y3 contains 3 mol% of yttrium and is also marketed as a free-flowing powder with (primary) particle size of 0.04 µm. Processing was similar as observed for TZ-0 powder. As with the pure zirconia grade, the polyurethane compositions tended to show brittle failure during tensile testing, especially at 60 mass% loading. The particle size distributions of the TZ-0 and TZ-Y3 grades as determined and shown in Figure 6, confirm that the particles are much larger than their primary particles; with maxima at about 60 and 50 µm, respectively, and a smaller maximum around 1 µm. This again suggests that, if these particles would be pretreated and/or (partly) deagglomerated before mixing with polymer, and/or a dispersing aid would be added during mixing, the observed processing and brittleness issues can be overcome. Also applying mixing equipment that allows higher forces than the small lab-scale apparatus used may enhance dispersion.

### Experiments 9 - 11.

In these examples medical grade zirconium oxide obtained from Sigma Aldrich (designated herein as ZrO/SA) was used, after having been dried at 120 °C during 24 h. These particles were found to have particle size D₅₀ of about 1.8 µm; which is much smaller than found for the particles used in above experiments 5-8 as also shown by Figure 6. Compounding was done analogously, but some additional mixing elements were used in the feeder to ensure proper dosing of the dried, somewhat sticky powder. Polymer strands and granules were made in a stable process. The compositions made showed an increase in tensile modulus with zirconia loading (20, 40 and 60 mass%), but a decrease in tensile strength and elongation. All samples show a tensile properties profile (also in conditioned state) that would be suitable for use of the compositions in making a bone anchor. Addition of zirconia increased hardness from 75.7 ShD (Exp. 1) to 81.1 and 80.2 ShD (examples 10 and 11)

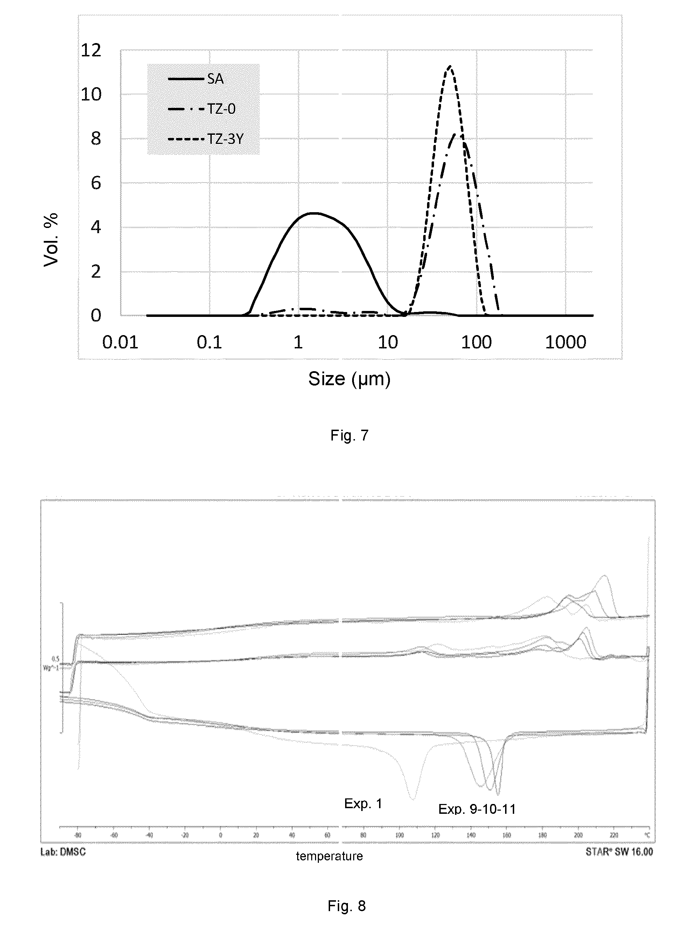

GPC measurements indicate a lowering of molar mass upon melt processing, especially during compounding; but these reductions appear to be significantly less than as observed for compositions comprising HA particles. The unfilled polyurethane shows higher molar mass after injection molding; but this material has not been subjected to a preceding compounding step. Crystallization behavior of selected samples was investigated with conventional DSC; relevant results (temperatures and enthalpies; J/gram sample) are summarized in Table 2 and DSC curves for experiments 1 and 9-10-11 are shown in Figure 7. For the composition TPU/HA 80/20 (Exp. 2) crystallization appears to start during cooling at higher temperature than for the reference polyurethane (Exp. 1), which may be related to low molar mass of degraded polyurethane and/or to some nucleation effect of hydroxyapatite particles. On the other hand, broadening of crystallization and melting peaks, specifically seen for higher amounts of filler particles, would point to hindering of polymer crystallization (lower rate). The compositions comprising zirconia particles show a nucleation effect in cooling, and higher melting temperatures in reheating scans.

### Cartilage plugs

Prototypes cartilage plugs having a mushroom-type shape as shown in Fig. 2A-B were made on a bench-top Xplore IM12 micro injection moulder, using a mold holder part containing a 2-part mold and applying a set of inserts to enable 2-component molding.

Materials used were the TPU composition comprising 60 mass% zirconia (Exp. 11) and an unfilled, end-group modified polycarbonate urethane of hardness 80 ShA (Bionate^{®} II 80A; DSM Biomedical BV, Sittard-Geleen, NL). Materials were dried before use during 24 h at 120 °C or during 72 h at 80 °C, respectively, under vacuum/nitrogen flush.

The different mold parts together define an implant part with following basic dimensions: a stem section with length 6.5 mm, diameter 6.1 mm, and bottom end rounded at 45° angle; a cap section with diameter 10.1 mm, top surface contoured with two radii of 11 and 18 mm rectangular to each other, with a total cap height of 3.5 mm of which the lower 1.0 mm connecting to the stem to be made from the rigid polymer composition. All further edges were rounded with a radius of 0.5 mm. The surface of the mold parts defining the stem section was etched to result in a texture with roughness VDI3400 36.

| **Table 1** | | | | | | | | | | |
|---|---|---|---|---|---|---|---|---|---|---|
| *Experiment* | *Filler particles* | | Tensile *properties* | | | | | | *Molar* mass *(Mw)* | |
| | *Type* | *Amount (mass%)* | *E-modulus (MPa)* | | *Tensile strength (MPa)* | | *Elongation at break* (%) | | *Pellets (kDa)* | *Bars (kDa)* |
| | | | *Dry*/*20 °C* | *Wet*/*37 °C* | *Dry*/*20* °C | *Wet*/*37 °C* | *Dry*/*20 °C* | *Wet* /*37 °C* | | |
| 1 | None | 0 | 1492 ± 49 | 207 ± 20 | 68 ± 13 | 44 ± 6 | 158 ± 57 | 162 ± 41 | 388 | 221 |
| 2 | HA | 20 | 1333 ± 55 | 438 ± 40 | 25 ± 8 | 2.9 ± 1.9 | 3.7 ± 3.4 | 2.9 ± 1.9 | | |
| 3 | HA | 40 | 1670 ± 600 | 445 ± 99 | 8.4 ± 5 | 2.5 ± 0.6 | 0.5 ± 0.2 | 0.8 ± 0.3 | 49 | 41 |
| 5 | ZrO/TZ-0 | 20 | 886 ± 57 | 286 ± 41 | 39 ± 4 | 26 ± 2 | 111 ± 14 | 192 ± 8 | | |
| 6 | ZrO/TZ-0 | 40 | 1180 ±68 | 433 ± 30 | 18± 5 | 7.8 ± 5.4 | 1.7 ± 0.5 | 4.5 ± 4.2 | | |
| 7 | ZrO/TZ-3Y | 40 | 1256 ± 63 | 312 ± 37 | 11 ± 4 | 8.8 ± 1.7 | 0.9 ± 0.4 | 4.6 ± 2.1 | | |
| 8 | ZrO/TZ-3Y | 60 | | 453 ± 34 | | 2.1 ± 1.1 | | 0.6 ± 0.3 | | |
| 9 | ZrO/SA | 20 | 945 ± 45 | 249 ± 18 | 51 ± 2 | 36 ± 1 | 119 ± 12 | 163 ± 13 | 162 | 124 |
| 10 | ZrO/SA | 40 | 1174 ± 27 | 326 ± 30 | 41 ± 2 | 24 ± 3 | 81 ± 25 | 139 ± 24 | | |
| 11 | ZrO/SA | 60 | 1435 ± 35 | 480 ± 24 | 36 ± 1 | 16 ± 1 | 10 ± 2 | 15 ± 11 | 100 | 79 |

**Table 2**

| *Experiment* | *Filler particles* | | *Thermal transitions observed in DSC curves* | | | | | |
|---|---|---|---|---|---|---|---|---|
| | *Type* | *Amount (mass%)* | *1^{rst} heating* | | *Cooling* | | *2^{nd} heating* | |
| | | | *°C* | *J*/*g* | °C | *J*/*g* | °*C* | *J*/*g* |
| 1 | None | 0 | 181 | 24 | 108 | -18 | 182 | 18 |
| | | | | | | | 204 | - |
| 2 | HA | 20 | 200 | 21 | 209 | -2 | 184 | 16 |
| | | | 216 | 1 | 148 | -16 | | |
| 6 | ZrO/TZ-0 | 40 | 177 | - | 151 | -17 | 199 | 16 |
| | | | 201 | 17 | | | | |
| | | | 216 | 1 | | | | |
| 7 | ZrO/TZ-3Y | 40 | 174 | - | 140 | -17 | 192 | 18 |
| | | | 200 | 24 | | | | |
| | | | 217 | 2 | | | | |
| 9 | ZrO/SA | 20 | 183 | - | 146 | -24 | 199 | - |
| | | | 191 | - | | | 215 | 21 |
| | | | 205 | 22 | | | | |
| 10 | ZrO/SA | 40 | 180 | - | 151 | -16 | 195 | - |
| | | | 189 | - | | | 209 | 16 |
| | | | 203 | 17 | | | | |
| 11 | ZrO/SA | 60 | 178 | - | 155 | -10 | 154 | - |
| | | | 201 | 15 | | | 193 | 10 |
| | | | 218 | 1 | | | | |

In a first step the polyurethane/zirconia composition was injected into the mold that comprised an insert in the cap section, applying a barrel temperature of 210 °C, a mold temperature of 80 °C, injection pressure 10 bar during 2.2 s, and packing pressure of 10 bar during 10 s. Then the insert was removed from the mold, and a tantalum wire of 4 mm length, which was pre-heated on a hot plate set at 250 °C, was placed in the mold on the injected surface, oriented with the 18 mm radius of the cap. Subsequently, the resilient polyurethane material was injected to the partly filled mold applying a barrel temperature of 235 °C, a mold temperature of 80 °C, injection pressure of 12 bar during 2.2 s, and packing pressure of 12 bar during 10 s to form the top section of the cap.

The molded plugs were functionalized with bioceramic particles by coating the stem and underside of the cap with a dispersion of BCP particles (biphasic calcium phosphate; Cam BioCeramics) in THF; air drying the sample, multiple rinsing with ethanol and drying (50 °C under reduced pressure). These plugs according to the present invention are designated as Plugs I).

Analogously to the above procedure, plugs were molded from corresponding unfilled materials, that is the stem section from a polycarbonate urethane of 75 ShD hardness (Bionate^{®} 75D; DSM Biomedical BV, Sittard-Geleen NL) and the cap section from the Bionate^{®} II 80A material. These unfilled plugs are hereinafter referred to as Plugs U.

In addition, metal plugs with geometry and dimensions corresponding to the polymeric plugs were made, wherein the stem part consists of titanium, the cap part is made from Cobalt-chromium, and stem was post-treated by corundum-blasting (designated as Plugs M).

### In vivo tests

The above-described 3 types of plugs were evaluated in an animal study, by implanting devices in the knees of 326 female Dutch milk goats. The study was approved by the local and national Animal Ethical Committee under project license PV2015-018-003.

The animal's knees (32 x 2) were divided in 4 groups, of which 3 were implanted with each 16 Plugs I, U and M; and the remaining group was sham-operated (placebo operated, without placing a plug) as a reference for natural cartilage degeneration.

The surgical procedure included making a medial parapatellar skin incision, opening the joint capsule to expose the medial femoral condyle, and locating the center of the weight bearing part thereof. For implanting a plug, an osteochondral defect was created using a cannulated drill under K-wire guidance. Depth of the drilled hole was controlled such that implants would be flush or somewhat recessed to the adjacent cartilage. Plugs were then inserted using press-fit fixation, while aligning the double curvature of the cap, using the orientational marker, to correspond with the knee morphology. No intraoperative or postoperative complications occurred.

After a 26-week (6 months) period at the facility for experimental animal testing of Maastricht University 4 animals of each group were euthanized with an overdose of pentobarbital (200 mg/kg bodymass). The knees were excised and subsequently dissected. The medial femoral condyles and tibia plateaus were isolated and fixed in neutral buffered formalin. Fixed specimens were dehydrated by incubation in increasing concentrations of ethanol in water, up to 100% ethanol. Medial femoral condyles were embedded in a hydroxyethyl methacrylate-based resin (Technovit 8100, Hereaus Kulzer, Hanua, DE) under vacuum. A polymethyl methacrylate (PMMA, Technovit 3040, Hereaus Kulzer, Hanua, DE) mantle was subsequently created for each block to prevent swelling. Plastic blocks then were cut using a band saw to orient the implants horizontally, and blocks were mounted in a diamond saw (SP1600, Leica Biosystems, Nussloch, DE) using ultra-low viscosity cyanoacrylate glue. A cut through the middle of the implant was made. Safranin-O/Fast-Green (Carl Roth, Karlsruhe, DE) staining was applied. Tissues were gently wiped dry and allowed to air dry for five to ten minutes. A glass coverslip was glued to the tissue using cyanoacrylate glue. Sections of 50-70 µm were cut and glued to a glass slide with cyanoacrylate glue. The sections were scanned using bright light microscopy at a magnification of 200x (M8 Microscope, Precipoint, Freising, DE). A custom written MATLAB script (MathWorks, Natick MA, US) was used to determine the bone-implant-contact (BIC), defined as the percentage of the implant surface that is in direct contact with bone.

The remaining 16 animals were euthanized 12 months after operation, and the knees and implants were evaluated using the procedures as described above.

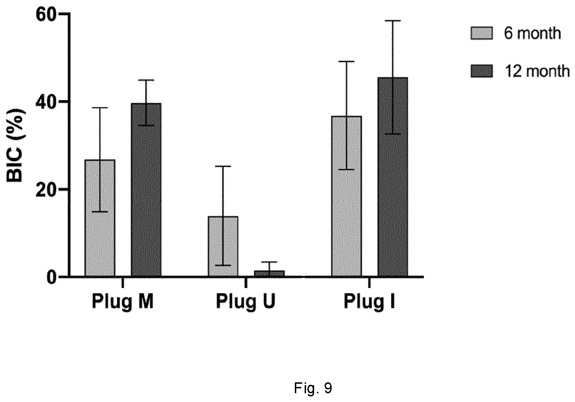

Results of BIC scoring of the implants after 6 and 12 months, expressed as averaged numeric score (%), are shown in Figure 9. It may be observed that the Plugs I, having a stem made from zirconia-TPU composite material and provided with BCP particles on the surface, showed the highest average BIC score; indicative of better bone-to-implant contact or in vivo osteointegration of this type of plug than as observed for plugs M and U. It was noted that one plug M and one plug I showed hardly any bone contacting after 6 months; which appeared to be caused by misalignment or tilting of the metal plug, and by cracking initiated e.g. at air inclusions in the plug based on the filled polymer composition. 4 out of 8 of the implants wholly made from unfilled polyurethane had a very low BIC score, likely caused by deformations of the relatively soft plug upon load.

After 12 months of implantation, 1 of 8 plugs M, 4 of 8 plugs I, and 8 of 8 plugs U had a BIC score of (nearly) 0 %. Again the unfilled plugs appear to lack sufficient stiffness, and in case of metal and composite plugs the low osseointegration score appears to origin from defects caused by the small scale compounding and/or molding processes that were applied or by the method of plug insertion during operation; rather than from actual performance of a plug that was properly fabricated and placed. The other plugs M and I both performed well, the photographs showing an increase in bone bonding relative to the results at 6 months. For the metal plugs this was not unexpected, as it is known that initially a metal plug will show relatively slow bonding to bone, but after several years the so-called stress shielding effect generally causes debonding. If the mentioned erroneous results are disregarded, the BIC scores for M and I plugs at the 12 month timepoint (as shown in Fig. 9) are on the order of 35-60 %, with the average score for the plugs I being higher (46±13 vs 40±5 %). It is anticipated that if the production of the polymer composition and molding of composite plugs I is up-scaled and improved, and implantation of plugs is better controlled with optimized tools, the observed defects and negative results may be reduced or even prevented.

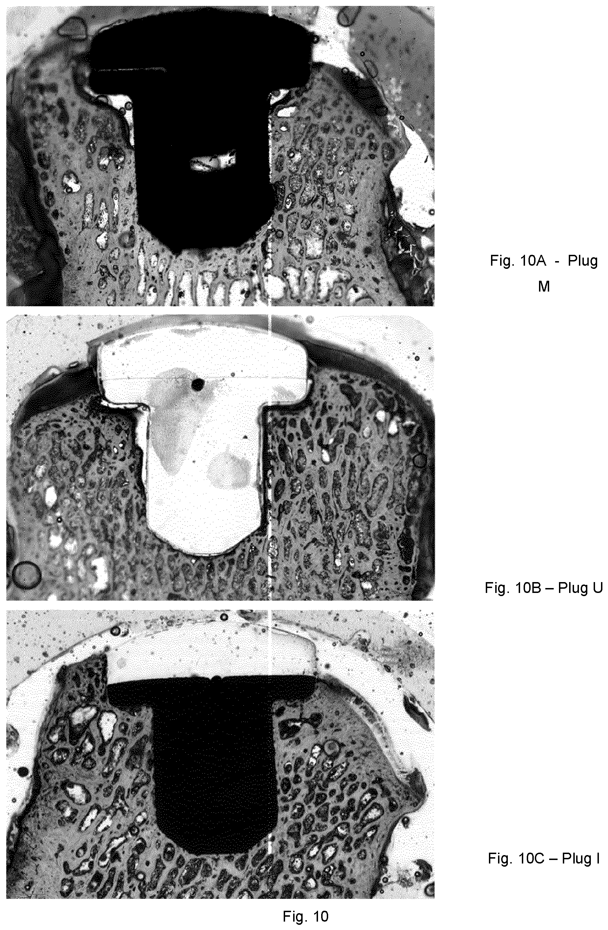

The above is further illustrated by representative photos of histological slices as shown in Figures 10 for each of the plug types (after 6 months, and using transmitting light). It is noted that these photos are grayscale versions of the original color photos. Nevertheless, it can be unambiguously concluded that the plug I shows close fitting to surrounding tissue, without voids or other irregularities at the interface or in the tissue. Note also the clearly visible distinction between the top cartilage replacing part and the bone anchoring part of the TPU-based plugs, and presence of a tantalum orientation marker (shown as a dark dot, the elongated marker being oriented perpendicular to the slice) at the interface of both parts.

It was further noted that MRI images taken of knees with implants after 6 m of implantation showed no substantial differences between Plugs I and U, although images of Plugs I showed better contrast between plug and tissue. Both plugs appeared not to interfere with MRI assessment of the cartilage in the joint, whereas MRI imaging of metal plugs M and adjacent cartilage was not feasible due to artefacts caused by the metal parts. The stem part of Plugs I, like Plugs M, were also well visible on X-ray photographs, but Plugs U were only partly and faintly visible.

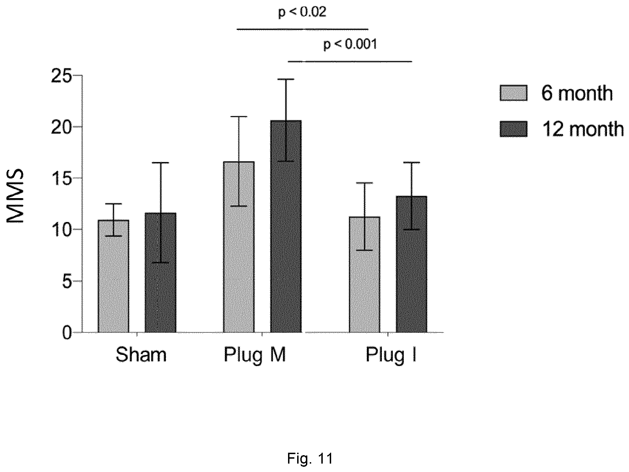

After fixation and dehydration of the tibia plateaus, 3-4 mm thick coronal osteochondral slabs were cut from the tibia plateau using a band saw; after 6 and 12 month periods. Individual slabs were then decalcified in formic acid at room temperature during at least six weeks. Specimen were subsequently embedded in paraffin, and 5 µm thick sections were cut using a standard microtome (Leica RM2245, Leica Biosystems, Nussloch, DE). Slices were stained with Safranin-O/Fast-Green (Carl Roth, Karlsruhe, DE) and subsequently digitized using a bright light microscopy slide scanner at a magnification of 200x (M8 Microscope, Precipoint, Freising, DE). Finally, scoring of tibia cartilage quality was performed using the Modified Mankin scoring (MMS) system, as described by Little et al. (DOI: 10.1016/j.joca.2010.04.016). In Figure 11 the results of numerically scoring cartilage damage or degeneration on the opposing bone surface is summarized. In these cases, the metal implants were found to have caused substantially more severe cartilage damage than the TPU-based plugs and the sham-operated group. Plugs I, having a zirconia-filled polyurethane stem and a cartilage contacting part of unfilled polyurethane, were found to perform similarly to the sham-operated goats, that is to goats having only natural cartilage in their knee joint. Plugs U scored similarly as plugs I, the soft polyurethane not having caused damage; but are not shown in figure 11 for simplicity reasons.

In summary, the cartilage plugs having a stem made from a zirconia/TPU composition and a cartilage replacing cap made from unreinforced TPU (Plugs I) showed best overall performance; with proper handleability and implantability, showing good osseointegration, causing little damage on the opposing cartilage surface, and being imageable as an implant with micro-CT, X-ray and MRI techniques. In addition, in this disclosure also several options are described to further improve the properties of filled TPU compositions, the design of cartilage plugs made therewith, and thus the performance of such implants.

## Claims

1. An orthopedic implant (1) having a bone anchoring part (3) comprising a polymer composition comprising a biostable thermoplastic polyurethane and 15-70 mass% of inorganic particles, **characterized in that** the inorganic particles comprise zirconia, wherein the inorganic particles have an average particle size in the range 0.03 - 10 µm, and wherein the implant further comprises a cartilage replacing part (2) that is made from a resilient, abrasion-resistant, biocompatible material.

2. The orthopedic implant according to claim 1, wherein the thermoplastic polyurethane of the bone anchoring part has a hardness of 50-90 ShD, preferably of 60-85 ShD.

3. The orthopedic implant according to claim 1 or 2, wherein the thermoplastic polyurethane of the bone anchoring part comprises an aliphatic polyether, an aliphatic polyester, an aliphatic polycarbonate or a combination thereof as soft block, preferably the thermoplastic polyurethane comprises an aliphatic polycarbonate.

4. The orthopedic implant according to any one of claims 1-3, wherein the inorganic particles comprising zirconia have an average particle size in the range 0.1-5 µm.

5. The orthopedic implant according to any one of claims 1-4, wherein the bone anchoring part has an outer surface having a surface texture with a roughness Ra of at least 1 µm.

6. The orthopedic implant according to any one of claims 1-5, wherein the bone anchoring part has bioactive particles or a bioactive coating on its surface, preferably comprising bioactive ceramic particles to induce osteoconductive properties.

7. The orthopedic implant according to any one of claims 1-6, wherein the cartilage replacing part is made from a biostable thermoplastic polyurethane comprising an aliphatic polyether, an aliphatic polyester, an aliphatic polycarbonate or a combination thereof as soft block, more preferably the thermoplastic polyurethane of the cartilage replacing part comprises an aliphatic polycarbonate.

8. The orthopedic implant according to claim 7, wherein the thermoplastic polyurethane of the cartilage replacing part has a hardness of 55-100 ShA, preferably of 70-90 ShA.

9. The orthopedic implant according to any one of claims 7-8, wherein the thermoplastic polyurethane of the cartilage replacing part comprises an average of 2 endgroups, preferably the endgroups are hydrophobic.

10. The orthopedic implant according to any one of claims 7-9, wherein the cartilage replacing part of the implant has a thickness of 0.2-5 mm, preferably of 0.8-3.4 mm.

11. The orthopedic implant according to any one of claims 7-10, wherein the cartilage replacing part has a contoured top surface with a curvature **characterized by** two different radii in directions rectangular to each other.

12. The orthopedic implant according to any one of claims 1-11, wherein the implant comprises a bone anchoring part and a cartilage replacing part, and wherein the bone anchoring part has a surface that contacts the cartilage replacing part, which surface is structured with protrusions and/or recessions of 0.05-3.0 mm in height and/or depth and in width.

13. The orthopedic implant according to any one of claims 1-12, wherein the implant further contains an orientation marker, preferably an elongated, radiopaque marker.

14. A method of making the orthopedic implant according to any one of claims 1-13 with a multi-component injection molding process, comprising a step of injecting the polymer composition comprising a biostable thermoplastic polyurethane and 15-70 mass% of inorganic particles comprising zirconia into a mold, which mold contains an insert in the form of the cartilage replacing part, to form the bone anchoring part, and subsequent steps of removing the insert from the mold and of injecting a resilient, abrasion-resistant, biocompatible material into the mold partly filled with the polymer composition to form the cartilage replacing part.

15. A surgical kit of parts comprising at least one orthopedic implant according to any of claims 1-13, preferably the kit comprises a set of at least two implants having different sizes, and optionally the kit further comprises auxiliary tools for preparing an implant location to receive an orthopedic implant and for inserting an orthopedic implant into a bone hole.

## Patentansprüche

1. Orthopädisches Implantat (1) mit einem Knochenverankerungsteil (3), der eine Polymerzusammensetzung umfasst, die ein biostabiles thermoplastisches Polyurethan und 15-70 Masse-% an anorganischen Partikeln umfasst, **dadurch gekennzeichnet, dass** die anorganischen Partikel Zirkoniumdioxid umfassen, wobei die anorganischen Partikel eine mittlere Partikelgröße in dem Bereich von 0,03-10 µm aufweisen und wobei das Implantat ferner einen Knorpelersatzteil (2) aus einem elastischen abriebfesten biokompatiblen Material umfasst.

2. Orthopädisches Implantat nach Anspruch 1, wobei das thermoplastische Polyurethan des Knochenverankerungsteils eine Härte von 50-90 ShD, vorzugsweise von 60-85 ShD, aufweist.

3. Orthopädisches Implantat nach Anspruch 1 oder 2, wobei das thermoplastische Polyurethan des Knochenverankerungsteils einen aliphatischen Polyether, einen aliphatischen Polyester, ein aliphatisches Polycarbonat oder eine Kombination davon als weichen Block umfasst, wobei das thermoplastische Polyurethan vorzugsweise ein aliphatisches Polycarbonat umfasst.

4. Orthopädisches Implantat nach einem der Ansprüche 1-3, wobei die anorganischen Partikel, die Zirkoniumdioxid umfassen, eine mittlere Partikelgröße in dem Bereich von 0,1-5 µm aufweisen.

5. Orthopädisches Implantat nach einem der Ansprüche 1-4, wobei der Knochenverankerungsteil eine Außenfläche mit einer Oberflächentextur mit einer Rauigkeit Ra von wenigstens 1 µm aufweist.

6. Orthopädisches Implantat nach einem der Ansprüche 1-5, wobei der Knochenverankerungsteil bioaktive Partikel oder eine bioaktive Beschichtung auf seiner Oberfläche, vorzugsweise umfassend bioaktive Keramikpartikel, aufweist, um osteokonduktive Eigenschaften zu induzieren.

7. Orthopädisches Implantat nach einem der Ansprüche 1-6, wobei der Knorpelersatzteil aus einem biostabilen thermoplastischen Polyurethan einen aliphatischen Polyether, einen aliphatischen Polyester, ein aliphatisches Polycarbonat oder eine Kombination davon als weichen Block umfasst, wobei das thermoplastische Polyurethan des Knorpelersatzteils bevorzugter ein aliphatisches Polycarbonat umfasst.

8. Orthopädisches Implantat nach Anspruch 7, wobei das thermoplastische Polyurethan des Knorpelersatzteils eine Härte von 55-100 ShA, vorzugsweise von 70-90 ShA, aufweist.

9. Orthopädisches Implantat nach einem der Ansprüche 7-8, wobei das thermoplastische Polyurethan des Knorpelersatzteils im Mittel 2 Endgruppen umfasst, wobei die Endgruppen vorzugsweise hydrophob sind.

10. Orthopädisches Implantat nach einem der Ansprüche 7-9, wobei der Knorpelersatzteil des Implantats eine Dicke von 0,2-5 mm, vorzugsweise von 0,8-3,4 mm, aufweist.

11. Orthopädisches Implantat nach einem der Ansprüche 7-10, wobei der Knorpelersatzteil eine konturierte obere Oberfläche mit einer Krümmung aufweist, die durch zwei unterschiedliche Radien in zueinander senkrechten Richtungen gekennzeichnet ist.

12. Orthopädisches Implantat nach einem der Ansprüche 1-11, wobei das Implantat einen Knochenverankerungsteil und einen Knorpelersatzteil umfasst und wobei der Knochenverankerungsteil eine Oberfläche aufweist, die mit dem Knorpelersatzteil Kontakt bildet, wobei die Oberfläche mit Vorsprüngen und/oder Vertiefungen von 0,05-3,0 mm Höhe und/oder Tiefe und Breite strukturiert ist.

13. Orthopädisches Implantat nach einem der Ansprüche 1-12, wobei das Implantat ferner einen Orientierungsmarker, vorzugsweise einen länglichen röntgendichten Marker, enthält.

14. Verfahren zur Herstellung des orthopädischen Implantats nach einem der Ansprüche 1-13 mit einem Mehrkomponenten-Spritzgussverfahren, umfassend einen Schritt des Einspritzens der Polymerzusammensetzung, die ein biostabiles thermoplastisches Polyurethan und 15-70 Masse-% an anorganischen Partikel umfassend Zirkoniumdioxid umfasst, in ein Formwerkzeug, wobei das Formwerkzeug einen Einsatz in der Form des Knorpelersatzteils enthält, um den Knochenverankerungsteil zu bilden, und nachfolgende Schritte des Entfernens des Einsatzes aus dem Formwerkzeug und des Einspritzens eines elastischen abriebfesten biokompatiblen Materials in das Formwerkzeug, das teilweise mit der Polymerzusammensetzung gefüllt ist, um den Knorpelersatzteil zu bilden.

15. Chirurgisches Kit, umfassend wenigstens ein orthopädisches Implantat nach einem der Ansprüche 1-13, wobei das Kit vorzugsweise einen Satz von wenigstens zwei Implantaten mit unterschiedlichen Größen umfasst und das Kit gegebenenfalls ferner Hilfswerkzeuge zum Vorbereiten einer Implantatstelle zur Aufnahme eines orthopädischen Implantats und zum Einführen eines orthopädischen Implantats in ein Knochenloch umfasst.

## Revendications

1. Implant orthopédique (1) ayant une partie d'ancrage osseux (3) comprenant une composition polymère comprenant un polyuréthane thermoplastique biostable et 15 à 70 % en masse de particules inorganiques, **caractérisé en ce que** les particules inorganiques comprennent de la zircone, dans lequel les particules inorganiques ont une taille moyenne de particule dans la plage de 0,03 à 10 µm, et dans lequel l'implant comprend en outre une partie de remplacement de cartilage (2) qui est faite d'un matériau biocompatible résilient et résistant à l'abrasion.

2. Implant orthopédique selon la revendication 1, dans lequel le polyuréthane thermoplastique de la partie d'ancrage osseux a une dureté de 50 à 90 ShD, de préférence de 60 à 85 ShD.

3. Implant orthopédique selon la revendication 1 ou 2, dans lequel le polyuréthane thermoplastique de la partie d'ancrage osseux comprend un polyéther aliphatique, un polyester aliphatique, un polycarbonate aliphatique ou une combinaison de ceux-ci comme bloc mou, de préférence le polyuréthane thermoplastique comprend un polycarbonate aliphatique.

4. Implant orthopédique selon l'une quelconque des revendications 1 à 3, dans lequel les particules inorganiques comprenant de la zircone ont une taille moyenne de particule dans la plage de 0,1 à 5 µm.

5. Implant orthopédique selon l'une quelconque des revendications 1 à 4, dans lequel la partie d'ancrage osseux a une surface externe ayant une texture de surface avec une rugosité Ra d'au moins 1 µm.

6. Implant orthopédique selon l'une quelconque des revendications 1 à 5, dans lequel la partie d'ancrage osseux a des particules bioactives ou un revêtement bioactif sur sa surface, comprenant de préférence des particules céramiques bioactives pour induire des propriétés ostéoconductrices.

7. Implant orthopédique selon l'une quelconque des revendications 1 à 6, dans lequel la partie de remplacement de cartilage est fabriquée à partir d'un polyuréthane thermoplastique biostable comprenant un polyéther aliphatique, un polyester aliphatique, un polycarbonate aliphatique ou une combinaison de ceux-ci comme bloc mou, plus préférablement le polyuréthane thermoplastique de la partie de remplacement de cartilage comprend un polycarbonate aliphatique.

8. Implant orthopédique selon la revendication 7, dans lequel le polyuréthane thermoplastique de la partie de remplacement de cartilage a une dureté de 55 à 100 ShA, de préférence de 70 à 90 ShA.

9. Implant orthopédique selon l'une quelconque des revendications 7 à 8, dans lequel le polyuréthane thermoplastique de la partie de remplacement de cartilage comprend une moyenne de 2 groupes terminaux, de préférence les groupes terminaux sont hydrophobes.

10. Implant orthopédique selon l'une quelconque des revendications 7 à 9, dans lequel la partie de remplacement de cartilage de l'implant a une épaisseur de 0,2 à 5 mm, de préférence de 0,8 à 3,4 mm.

11. Implant orthopédique selon l'une quelconque des revendications 7 à 10, dans lequel la partie de remplacement de cartilage a une surface supérieure profilée avec une courbure **caractérisée par** deux rayons différents dans des directions rectangulaires l'une par rapport à l'autre.

12. Implant orthopédique selon l'une quelconque des revendications 1 à 11, dans lequel l'implant comprend une partie d'ancrage osseux et une partie de remplacement de cartilage, et dans lequel la partie d'ancrage osseux a une surface qui est en contact avec la partie de remplacement de cartilage, laquelle surface est structurée avec des protubérances et/ou des creux de 0,05 à 3,0 mm de hauteur et/ou profondeur et de largeur.

13. Implant orthopédique selon l'une quelconque des revendications 1 à 12, dans lequel l'implant contient en outre un marqueur d'orientation, de préférence un marqueur radio-opaque allongé.

14. Procédé de fabrication de l'implant orthopédique selon l'une quelconque des revendications 1 à 13 avec un procédé de moulage par injection à plusieurs composants, comprenant une étape d'injection de la composition polymère comprenant un polyuréthane thermoplastique biostable et 15 à 70 % en masse de particules inorganiques comprenant de la zircone dans un moule, lequel moule contient un insert sous la forme de la partie de remplacement de cartilage, pour former la partie d'ancrage osseux, et les étapes suivantes de retrait de l'insert du moule et d'injection d'un matériau biocompatible résilient et résistant à l'abrasion dans le moule partiellement rempli de la composition polymère pour former la partie de remplacement de cartilage.

15. Kit chirurgical de parties comprenant au moins un implant orthopédique selon l'une quelconque des revendications 1 à 13, de préférence le kit comprend un ensemble d'au moins deux implants ayant des tailles différentes, et facultativement le kit comprend en outre des outils auxiliaires pour préparer un emplacement d'implant pour recevoir un implant orthopédique et pour insérer un implant orthopédique dans un trou osseux.
